# EUROPÄISCHE PATENTSCHRIFT

(11) **EP 2 648 689 B1**
(45) Veröffentlichungstag und Bekanntmachung des Hinweises auf die Patenterteilung: **20.12.2017**
(21) Anmeldenummer: 11791435.8
(22) Anmeldetag: 14.11.2011
(51) Int. Cl.: A61K 8/49, A61Q 19/06, A61Q 19/08, C07D 309/36, C07D 309/38

(54) **2-PYRONE**
2-PYRONES
2-PYRONES

(30) Priorität: 10.12.2010 DE 102010054149
(43) Veröffentlichungstag der Anmeldung: 16.10.2013
(73) Patentinhaber: Merck Patent GmbH, 64293 Darmstadt (DE)
(72) Erfinder: CAROLA, Christophe, 64625 Bensheim (DE); MUELLER, Tatjana, 64846 Gross-Zimmern (DE)
(86) Internationale Anmeldenummer: PCT/EP2011/005729
(87) Internationale Veröffentlichungsnummer: WO 2012/076109

(56) Entgegenhaltungen:
- EP-A1- 0 841 063
- FR-A1- 2 774 905
- DATABASE WPI Week 200834 Thomson Scientific, London, GB; AN 2008-E99132 XP002697567, & WO 2007/125832 A1 (FANCL CORP) 8. November 2007 (2007-11-08)

## Beschreibung

Die vorliegende Erfindung betrifft die nicht-therapeutische Verwendung von Verbindungen der Formel (I) zur Pflege, Konservierung oder Verbesserung des allgemeinen Zustandes oder des Erscheinungsbildes der Haut oder Haare, sowie Zubereitungen enthaltend Verbindungen der Formel (I).

Die menschliche Haut unterliegt gewissen Alterungsprozessen, die teilweise auf intrinsische Prozesse (chronoaging) und teilweise auf exogene Faktoren (environmental, z.B. photoaging) zurückzuführen sind. Zusätzlich können vorübergehende oder auch andauernde Veränderungen des Hautbildes auftreten, wie Akne, fettige oder trockene Haut, Keratosen, Rosaceae, lichtempfindliche, entzündliche, erythematöse, allergische oder autoimmunreaktive Reaktionen wie Dermatosen und Photodermatosen.

Zu den exogenen Faktoren zählen insbesondere das Sonnenlicht oder künstliche Strahlungsquellen mit vergleichbarem Spektrum sowie Verbindungen, die durch die Strahlung entstehen können, wie undefinierte reaktive Photoprodukte, die auch radikalisch oder ionisch sein können. Zu diesen Faktoren zählen auch Zigarettenrauch und die darin enthaltenen reaktiven Verbindungen wie Ozon, freie Radikale, beispielsweise das Hydroxylradikal, Singulettsauerstoff und andere reaktive Sauerstoff- oder Stickstoffverbindungen, die die natürliche Physiologie oder Morphologie der Haut stören.

Durch den Einfluss dieser Faktoren kann es unter anderem zu direkten Schäden an der DNA der Hautzellen kommen sowie an den Kollagen-, Elastin- oder Glycosaminoglycanmolekülen der extrazellulären Matrix, die für die Festigkeit der Haut verantwortlich sind. Darüber hinaus kann es zu einer Beeinflussung der Signaltransduktionsketten kommen, an deren Ende die Aktivierung matrixabbauender Enzyme steht. Wichtige Vertreter dieser Enzyme sind die Matrixmetalloproteinasen (MMPs, z.B. Kollagenasen, Gelatinasen, Stromelysine), deren Aktivität zusätzlich durch TIMPs (tissue inhibitor of matrix metalloproteinases) reguliert wird.

Die Folgen der o.g. Alterungsprozesse sind Verdünnung der Haut, schwächere Verzahnung von Epidermis und Dermis, Reduktion der Zellzahl sowie der versorgenden Blutgefäße. Dabei kommt es zur Ausbildung von feinen Linien und Falten, die Haut wird ledrig und es können Pigmentstörungen auftreten.

Die gleichen Faktoren wirken auch auf Haare, wo es ebenfalls zu einer Schädigung kommen kann. Die Haare werden spröde, weniger elastisch und glanzlos. Die Oberflächenstruktur der Haare ist geschädigt.

Kosmetische oder dermatologische Pflegeprodukte mit Eigenschaften, die den oben beschriebenen oder vergleichbaren Prozessen entgegenwirken oder deren schädliche Folgen mindern oder rückgängig machen sollen, zeichnen sich häufig durch folgende spezifische Eigenschaften aus - radikalfangend, antioxidativ, entzündungshemmend oder feuchthaltend wirksam. Sie verhindern oder reduzieren u.a. die Aktivität der matrixabbauenden Enzyme oder regulieren die Neusynthese von Kollagen, Elastin oder Proteoglycanen.

Die Verwendung von Antioxidantien oder Radikalfängern in kosmetischen Zubereitungen ist an sich hinlänglich bekannt. So ist der Einsatz des antioxidativen Vitamin E in Sonnenschutzformulierungen üblich. Dennoch bleibt auch hier die erzielte Wirkung hinter der erhofften weit zurück.

Vitamin A und Vitamin-A-Derivate, wie Retinsäure, Retoinol und Retinol-Ester, wirken auf die Differenzierung von Epithelzellen und werden daher zur Prophylaxe und Behandlung zahlreicher den Hautzustand beeinträchtigender Phänomene eingesetzt; z.B. ist die Verwendung gegen Akne, Psoriaris, Altersflecken, Hautverfärbungen und Falten beschrieben. (vgl. z.B. WO 93/19743, WO 02/02074).

Cellulite bezeichnet die Dellenbildung der Haut, die bei vielen Frauen hauptsächlich im Bereich der Oberschenkel, des Gesäßes, aber auch am Bauch und den Oberarmen auftritt. Viele betroffene Frauen leiden psychisch unter diesem ästhetischen Problem. Auch wenn die Ursache der Cellulite bisher nicht vollständig bekannt ist, scheint sie hauptsächlich auf die lokale Anhäufung von Fett und die anatomische Beschaffenheit des subkutanen Fettgewebes zurückzuführen sein. Histologische Studien an subkutanem Fettgewebe von Frauen und Männern haben ergeben, dass die Fettläppchen bei Frauen größer und vertikaler angeordnet sind als bei Männern. Diese können sich nach außen gegen die Dermis ausdehnen und so die für Cellulite charakteristischen Dellen und Erhebungen erzeugen. Aufgrund der unterschiedlichen Verteilung von alpha- und beta-adrenergen Rezeptoren weisen die subkutanen Fettablagerungen der Oberschenkel der Frauen meist auch eine geringere lipolytische Aktivität auf als die Fettablagerungen am Bauch oder anderen Körperregionen. Die Steigerung der Lipolyse oder die Reduktion der Fettablagerungen an diesen speziellen Stellen kann daher dazu beitragen Cellulite zu reduzieren oder ihr vorzubeugen.

Die am besten bekannte und angewandte Methode zur Lipolyse-Stimulation besteht in der Hemmung der Phosphodiesterase, um den Abbau von cyclischem AMP (cAMP) zu unterdrücken oder wenigstens zu minimieren, da cAMP als Aktivator für die Lipolyse agiert. Bekannte Wirkstoffe für die topische Anwendung zur Behandlung von Cellulite durch Lipolyse sind beispielsweise Xanthinanaloga wie Theobromin, Aminophyllin, Coffein oder Theophyllin.

Weitere bekannte Methoden zur Behandlung von Cellulite basieren auf der Stimulierung der Adenylatcyclase zur Steigerung der cAMP-Konzentration (beta- adrenerge Agonisten) oder auf der Blockierung der antilipolytischen Inaktivierung der Adenylatcyclase (alpha-2-adrenerge Antagonisten). Beispielsweise ist Isoproterenol ein bekannter beta-adrenerger Stimulator (US 4588724 B1).

Weiterhin ist bekannt, dass auch die Verwendung von bestimmten öllöslichen Pflanzenextrakten einen Schlankheitseffekt hat. Beispiele für solche Pflanzenextrakte sind die Extrakte von Kletterefeu (Hedera helix), Arnica (Arnica montana), Rosmarin (Rosmarinus officinalis N), Ringelblume (Calendula officinalis), Salbei (Salvia officinalis N), Ginseng (Panax ginseng), Hypericum perforatum, Ruscus aculeatus, Filipendula ulmaria L und Ortosifon stamincus Benth.

Auch Retinoide können die Anzeichen von Cellulite reduzieren, wenn sie topisch aufgetragen werden (EP-A-866 693; US 5,051,449). Die Hautoberfläche wird dadurch ebener und glatter.

Aufgrund des immer größer werdenden Bedarfs an kosmetischen Wirkstoffen zur vorbeugenden Behandlung von menschlicher Haut und menschlicher Haare gegen Alterungsprozesse und schädigenden Umwelteinflüssen, war es Aufgabe der vorliegenden Erfindung, neue kosmetische Wirkstoffe bereitzustellen, die die bereits eingangs genannten Wirkungen zeigen und gut formulierbar sind.

Ziel der vorligenden Erfindung ist daher die Bereitstellung von Verbindungen zur Pflege, Konservierung oder Verbesserung des allgemeinen Zustands oder des Erscheinungsbildes der Haut oder Haare.

Überraschend wurde nun gefunden, dass bestimmte 2-Pyron-Derivate als Anti-Ageing- bzw. Anti-Cellulite-Wirkstoffe eingesetzt werden können.

Im Stand der Technik sind Pyronderivate für verschiedene Anwendungen bekannt.
DE 4231465 A1 beschreibt die Verwendung von verschiedenen Acyloxypyranonen, wie 4-Acetoxy-6-methyl-2H-2-pyranon oder 4-Butyroxy-6-methyl-2H-2-pyranon, als Aktivatoren für anorganische Perverbindungen, die als Bleichmittel in Waschmitteln eingesetzt werden können.

In EP 0672406 A1 werden Pyronderivate offenbart, die das Haarwachstum stimulieren und den Haarausfall bremsen. Genannt werden beispielsweise die Verbindungen 5,6-Dihydro-6-methyl-2H-pyran-2-on, 4-Methoxy-6-(2-phenylethenyl)2H-pyran-2-on und 4-Methoxy-6-[2-(4-methoxyphenyl)ethenyl]2H-pyran-2-on.

In EP 1167362 A1 werden Pyronderivate als wohlriechende Lactone offenbart, die als Spaltprodukte aus Duftvorläuferverbindungen entstehen können, welche in parfümierten Produkten wie Waschzusammensetzungen, Reinigungsprodukte oder Körperpflegeprodukte zum Einsatz kommen können.

Die Verwendung von verschiedenen Pyronderivaten als Schädlingsbekämpfungsmittel zur Behandlung von Pflanzen gegen Pilzbefall wird in GB 2410435 A offenbart.

JP 2006-206467 A beschreibt, dass bestimmte Pyronderivate zur Steigerung der Melanogenese verwendet werden können.

In JP 2004-024298 A werden Pyronderivate zur Verwendung in schäumenden Deodorantwirkstoffen für Aerosole offenbart.

WO 2007/125832 A1 beschreibt die Verwendung der Verbindung Helipyron A unter anderem als Radikalfänger für Singulettsauerstoff, als anti-Aging- und Antifalten-Wirkstoff oder als Feuchtigkeitsspender.

FR 2774905 A1 beschreibt die Verwendung von Verbindungen, die eine Pyrongruppierung enthalten, zur Lipolyse.

EP 0841063 A1 beschreibt Ketonderivate und ihre medizinische Anwendung. Pyronstrukturen sind nicht umfasst.

Ein erster Gegenstand der vorliegenden Erfindung ist die nicht-therapeutische Verwendung mindestens einer Verbindung der Formel (I) 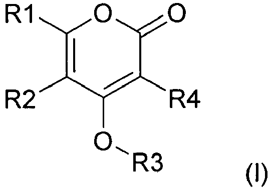 wobei
R1 für eine geradkettige oder verzweigte C₁- bis C₂₀-Alkylgruppe steht,
R2 steht für
   - H,
R4 steht für
   - H,
   - geradkettige oder verzweigte C₁- bis C₂₀-Alkylgruppe oder
   - geradkettige oder verzweigte C₂- bis C₂₀-Alkenylgruppe mit einer oder mehreren Doppelbindungen,
R3 steht für einen Rest ausgewählt aus
   - H,
   - geradkettige oder verzweigte C₁- bis C₂₀-Alkylgruppe,
   - geradkettige oder verzweigte C₂- bis C₂₀-Alkenylgruppe mit einer oder mehreren Doppelbindungen, wobei die Alkenylgruppe auch mit einer oder mehreren gesättigten oder ungesättigten C₃- bis C₁₂-Cycloalkylgruppen substituiert sein kann,
   - geradkettige oder verzweigte C₂- bis C₂₀-Alkinylgruppe mit einer oder mehreren Dreifachbindungen,
   - gesättigte oder ungesättigte C₃- bis C₁₂-Cycloalkylgruppe, wobei die Ringe jeweils auch durch -(CH₂)ₙ-Gruppen mit n = 1 bis 3 überbrückt sein können,
   - ein Acylrest der Formel -C(=O)-R6,
R6 steht für
   - geradkettige oder verzweigte C₁- bis C₂₀-Alkylgruppe,
   - einen Rest der Formel (II) 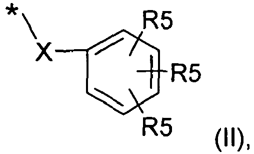 worin X für geradkettiges oder verzweigtes C₁- bis C₆-Alkylen oder geradkettiges oder verzweigtes C₂- bis C₆-Alkenylen steht und die Reste R5 unabhängig voneinander ausgewählt sind aus H, OH, geradkettiges oder verzweigtes C₁- bis C₆-Alkyl oder geradkettiges oder verzweigtes O-(C₁- bis C₆-Alkyl),
   zur Pflege, Konservierung oder Verbesserung des allgemeinen Zustands oder des Erscheinungsbildes der Haut oder Haare.

Bevorzugt werden die Verbindungen der Formel (I) zur Pflege, Konservierung oder Verbesserung des allgemeinen Zustands oder des Erscheinungsbildes der Haut verwendet.

Grundsätzlich sind im Sinne der vorliegenden Erfindung von der Bezeichnung "Verbindung nach Formel (I)" auch die Salze der Verbindungen nach Formel (I) umfasst. Zu den bevorzugten Salzen gehören dabei insbesondere Alkali- und Erdalkalimetallsalze sowie Ammonium-Salze, insbesondere jedoch Natrium- und Kalium-Salze.

In einer bevorzugten Ausführungsform der vorliegenden Erfindung wird die mindestens eine Verbindung der Formel (I) zur Vorbeugung, Reduzierung oder Bekämpfung von Cellulite oder von Anzeichen von Cellulite und/oder zur Reduzierung von lokaler Fettansammlung verwendet, besonders bevorzugt zur Stimulierung der Lipolyse.

Der Begriff Lipolyse bezeichnet hierbei die Mobilisierung von Fett in den Adipozyten des Fettgewebes durch hydrolytische Spaltung.

Die Verbindungen der Formel (I) sind beispielsweise dazu geeignet, das externe Erscheinungsbild der Haut und der Figur positiv zu beeinflussen. Durch Lipolyse kann neben der Reduzierung von Cellulite auch ein Verschlankungseffekt erreicht werden. Insgesamt führt dies dazu, dass die Haut glatter und straffer wirkt.
In einer weiteren bevorzugten Ausführungsform der vorliegenden Erfindung wird die mindestens eine Verbindung der Formel (I) zur Prophylaxe gegen oder Bekämpfung von zeit- und/oder lichtinduzierten Alterungsprozessen der Haut oder Haare verwendet.
Eine besonders bevorzugte Verwendung ist die Prophylaxe gegen oder Reduktion von Hautunebenheiten, wie Falten, feinen Linien, rauher Haut oder großporiger Haut.
Eine weitere besonders bevorzugte Verwendung der Verbindungen der Formel (I) ist die Verwendung zur Prophylaxe und/oder Verhinderung von vorzeitiger Hautalterung, insbesondere zur Prophylaxe und/oder Verhinderung von licht- oder alterungsbedingter Faltenbildung der Haut, zur Verminderung der Pigmentierungen und der Keratosis actinica im kosmetischen Sinne.
Erfindungsgemäß ist dabei die nicht-therapeutische Verwendung der Verbindungen der Formel (I).
Weiterhin können die Verbindungen der Formel (I) auch als Arzneimittelwirkstoff zur Prophylaxe und/oder Behandlung aller Krankheiten, die mit der normalen Alterung oder der licht-bedingten Alterung der Haut zusammenhängen, sowie zur Prophylaxe und/oder Behandlung von Hautkrankheiten, die mit einer Störung der Keratinisierung verbunden sind, die die Differenzierung und Zellproliferation betrifft, insbesondere zur Behandlung der Akne vulgaris, Akne comedonicá, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Haut-atopien, wie Ekzemen oder der respiratorischen Atopie oder auch der Hypertrophie des Zahnfleisches, sowie zur Prophylaxe und/oder Behandlung aller gutartigen oder bösartigen Wucherungen der Dermis oder Epidermis, die gegebenenfalls viralen Ursprungs sind, wie Verruca vulgaris, Veruca plana, Epidermodysplasia verruciformis, orale Papillomatose, Papillo-matosis florida, und der Wucherungen, die durch UV-Strahlung hervorgerufen werden können, insbesondere des Epithelioma baso-cellulare und Epithelioma spinocellulare, verwendet werden.

Die Verwendung von 2-Pyron-Derivaten der allgemeinen Formel (I) in Zubereitungen sowohl im kosmetischen Sinn als auch als Arzneimittelwirkstoff bietet u.a. einen Schutz vor Schäden, die durch UV-Strahlung oder durch reaktive Verbindungen hervorgerufene Prozesse direkt oder indirekt verursacht werden, wie z. B. der Hautalterung, dem Verlust der Hautfeuchtigkeit, dem Verlust der Hautelastizität, der Bildung von Falten oder Runzeln oder von Pigmentstörungen oder Altersflecken.
Weiterhin möglich ist die nicht-therapeutische Verwendung der o.g. Verbindungen oder deren Zubereitungen zur Vorbeugung unerwünschter Veränderungen des Hautbildes, wie z.B. Akne oder fettige Haut, Keratosen, lichtempfindliche, entzündliche, erythrematöse, allergische oder autoimmunreaktive Reaktionen im kosmetischen Sinne sowie die Verwendung als Arzneimittelwirkstoff für die genannten Veränderungen des Hautbildes. Verbindungen der Formel (I) können beispielsweise zur vorbeugenden Behandlungen von Entzündungen und Allergien der Haut sowie in bestimmten Fällen zur Verhütung bestimmter Krebsarten verwendet werden. Insbesondere eignen sich Verbindungen der Formel (I) als Arzneimittelwirkstoff zur Behandlung von Entzündungen, Allergien und Irritationen, insbesondere der Haut. Ferner können Zubereitungen hergestellt werden in einer Wirkung als Venentonikum, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel.

Die Verbindungen der Formel (I) bzw. Zubereitungen enthaltend mindestens eine Verbindung der Formel (I), dienen aber auch zur Beruhigung von empfindlicher und gereizter Haut, zur vorbeugenden Regulation der Kollagen-, Hyaluronsäure-, Elastinsynthese, Stimulation der DNA-Synthese, insbesondere bei defizitären oder hypoaktiven Hautzuständen, Regulation der Transkription und Translation matrixabbauender Enzyme, insbesondere der MMPs, Steigerung der Zellerneuerung und Regeneration der Haut, Steigerung der hauteigenen Schutz- und Reparaturmechanismen für DNA, Lipide und/oder Proteine.

In Formel (I) steht R1 bevorzugt für eine geradkettige oder verzweigte C₁-bis C₆-Alkylgruppe, besonders bevorzugt für Methyl.

R2 steht für H.

R4 steht bevorzugt für H oder eine geradkettige oder verzweigte C₂- bis C₂₀-Alkenylgruppe mit einer oder mehreren Doppelbindungen.
In einer besonders bevorzugten Ausführungsform der vorliegenden Erfindung steht R4 für eine geradkettige oder verzweigte C₂- bis C₂₀-Alkenylgruppe mit einer oder mehreren Doppelbindungen, insbesondere eine geradkettige oder verzweigte C₆- bis C₁₄-Alkenylgruppe mit einer oder mehreren Doppelbindungen.
In einer weiteren besonders bevorzugten Ausführungsform der vorliegenden Erfindung steht R4 für H.

Wenn R4 für H steht ist somit eine mögliche und bevorzugte Ausführungsform der vorliegenden Erfindung dadurch gegeben, dass die Verbindung der Formel (I) für eine Verbindung der Formel (III) 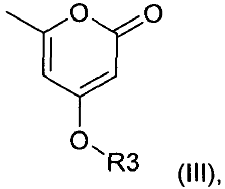 steht, worin R3 wie zuvor und nachfolgend definiert ist.

R3 steht bevorzugt für einen Rest ausgewählt aus
- H,
- geradkettige oder verzweigte C₁- bis C₂₀-Alkylgruppe,
- geradkettige oder verzweigte C₂- bis C₂₀-Alkenylgruppe mit einer oder mehreren Doppelbindungen, wobei die Alkenylgruppe auch mit einer oder mehreren gesättigten oder ungesättigten Cyclohexylgruppen substituiert sein kann,
- ein Acylrest der Formel -C(=O)-R6.

Die Reste R5 in Formel (I) sind bevorzugt unabhängig voneinander ausgewählt aus H, OH oder geradkettiges oder verzweigtes O-(C₁- bis C₆-Alkyl). Besonders bevorzugt sind die Reste R5 unabhängig voneinander ausgewählt aus H, OH und OCH₃. In einer ganz besonders bevorzugten Ausführungsform der vorliegenden Erfindung stehen zwei R5 für H und ein R5 für OCH₃.

In Formel (II) steht X bevorzugt für CH₂CH₂ oder CH=CH.

In einer besonders bevorzugten Ausführungsform der vorliegenden Erfindung ist die Verbindung der Formel (I) ausgewählt aus den Verbindungen der Formel (Ia) bis (Ij)

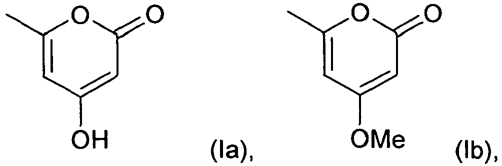

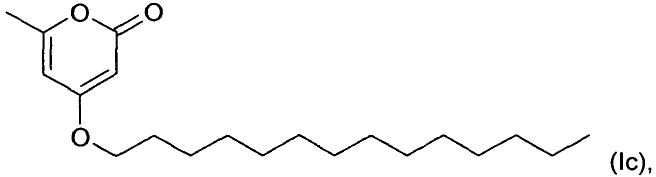

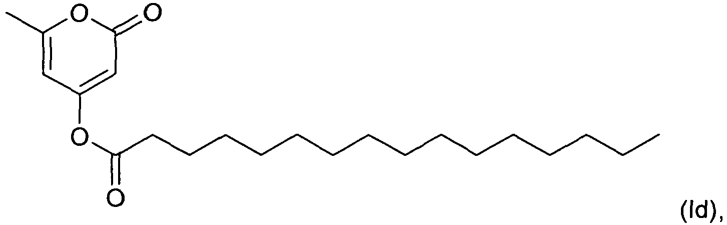

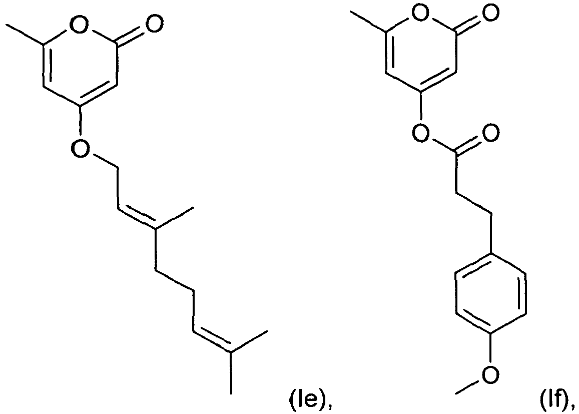

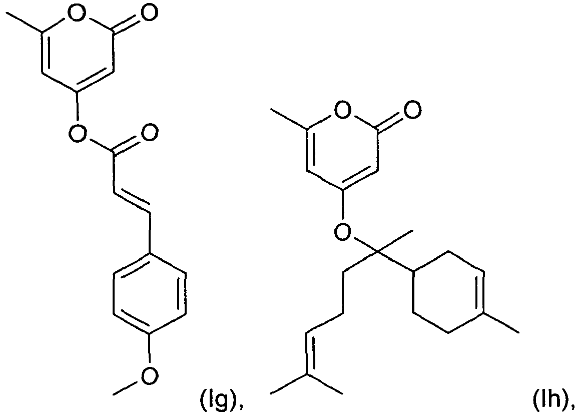

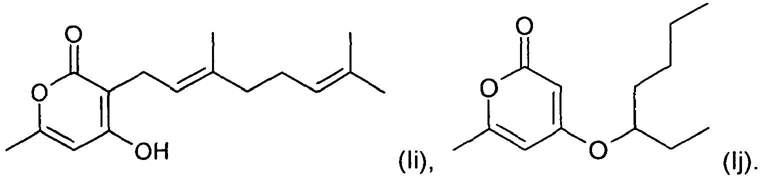

Für die Verwendung zur Prophylaxe gegen oder zur Bekämpfung von zeit- und/oder lichtinduzierten Alterungsprozessen der Haut oder Haare sind insbesondere die Verbindungen der Formel (Ia) und (Ii) vorteilhaft.

Für die Verwendung zur Vorbeugung, Reduzierung oder Bekämpfung von Cellulite oder von Anzeichen von Cellulite und/oder zur Reduzierung lokaler Fettansammlung sind insbesondere die Verbindungen der Formel (Ib) und (Ij) vorteilhaft.

Es wird vermutet, ohne an diese Theorie gebunden zu sein, dass die Verbindungen der Formel (I) durch das Zusammenspiel verschiedener Faktoren dazu beitragen, die Anzeichen von Cellulite zu verringern, zum Beispiel:
(1) Stimulierung der Fibroblasten zur Produktion größerer Mengen an Grundsubstanz (Glycoproteine und Glycosaminoglykane) in der die Kollagenfasern verteilt sind und die bei Hautdehnung aneinander vorbeigleiten.
(2) Steigerung der proliferativen und metabolischen Aktivität der Fibroblasten, wodurch neues Kollagen in der oberen Dermis abgelagert und die Haut verdichtet wird.
(3) Stimulierung des Blutflusses und Förderung der Bildung von Gefäßgewebe (Angiogenese), wodurch die Zirkulation und die Aktivität anderer Zellarten der Dermis verbessert wird.
Durch die Anwendung von Verbindungen der Formel (I) kann eine straffere, dickere und gesündere Dermis erreicht werden, die die Mobilität der leicht verformbaren Fettläppchen einschränkt und damit verhindert, dass diese aus der subkutanen Fettschicht in die darüberliegende Dermis herausragen.

Es wird vermutet, dass die Verbindungen der Formel I auch als Enzymhemmer wirken. Sie hemmen vermutlich Proteinkinasen, Elastase, Aldosereduktase sowie Hyaluronidase. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie vermutlich die AMP-Phosphodiesterase.

Aufgrund dieser Eigenschaften eignen sich die Verbindungen der Formel (I) allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse.

Insbesondere eignen sich die Verbindungen der Formel (I) auch zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonicá, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Haut-atopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillo-matosis florida, und der Wucherungen, die durch UV-Strahlung hervor-gerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Herz-/Kreislauf-Erkrankungen, wie Arteriosklerose oder Bluthochdruck, sowie des Insulin-unabhängigen Diabetes, zur Behandlung von Hautproblemen, die durch UV-Strahlung hervorgerufen werden.

Im Sinne der vorliegenden Erfindung handelt es sich bei einer geradkettigen oder verzweigten C₁- bis C₆-Alkylgruppe um einen Alkylrest mit 1 bis 6 C-Atomen, beispielsweise um Methyl, Ethyl, Isopropyl, Propyl, Butyl, sek.-Butyl oder tert.-Butyl, Pentyl, Isopentyl, 1-, 2- oder 3-Methylbutyl, 1,1-, 1,2- oder 2,2-Dimethylpropyl, 1-Ethylpropyl, 1-Ethyl-1-methylpropyl, 1-Ethyl-2-methylpropyl, 1,1,2-oder 1,2,2-Trimethylpropyl, 1,1-, 1,2-, 1,3-, 2,2-, 2,3- oder 3,3-Dimethylbutyl, 1- oder 2-Ethylbutyl, 1-, 2-, 3- oder 4-Methylpentyl oder Hexyl.
Bei einer C₁- bis C₂₀-Alkylgruppe kann es sich neben den oben gelisteten Resten beispielsweise auch um Heptyl, 1-Ethyl-pentyl, Octyl, 1-Ethyl-hexyl, Nonyl, Decyl, Undecyl, Dodecyl, Tridecyl, Tetradecyl, Pentadecyl, Hexadecyl, Heptadecyl, Octadecyl, Nonadecyl oder Eicosyl handeln.

Entsprechend handelt es sich bei einem C₁- bis C₆-Alkylenrest beispielsweise um Methylen, Ethylen, Propylen, Butylen, Pentylen oder Hexylen.

Erfindungsgemäß kann eine Alkenylgruppe eine oder mehrere Doppelbindungen enthalten. Eine geradkettige oder verzweigte C₂- bis C₂₀-Alkenylgruppe ist beispielsweise Allyl, Vinyl, Propenyl, 2- oder 3-Butenyl, Isobutenyl, sek.-Butenyl, 2-Methyl-1- oder 2-Butenyl, 3-Methyl-1-butenyl, 1,3-Butadienyl, 2-Methyl-1,3-butadienyl, 2,3-Dimethyl-1,3-butadienyl, 1-, 2-, 3- oder 4-Pentenyl, iso-Pentenyl, Hexenyl, Heptenyl oder Octenyl, -C₉H₁₇,-C₁₀H₁₉ bis -C₂₀H₃₉.

Beispiele für einen C₂- bis C₆-Alkenylenrest sind Ethenylen, Propenylen, 2- oder 3-Butenylen, 1-, 2-, 3- oder 4-Pentenylen oder Hexenylen.

Eine Alkinylgruppe kann eine oder mehrere Dreifachbindungen enthalten. Beispiele für eine verzweigte oder nicht verzweigte C₂- bis C₂₀-Alkinylgruppe sind Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇.

Eine C₃- bis C₁₂-Cycloalkylgruppe bezeichnet im Sinne der Erfindung gesättigte und teilweise ungesättigte nicht-aromatische cyclische Kohlenwasserstoffgruppen, die 3 bis 12 C-Atome umfassen und auch durch -(CH₂)ₙ-Gruppen, mit n=1, 2 oder 3, überbrückt sein können. Die Bindung an den jeweiligen Rest kann über jedes Ringmitglied der Cycloalkylgruppe erfolgen. Beispiele für geeignete Cycloalkylreste sind Cyclopropyl, Cyclobutyl, Cyclopentyl, Cyclohexyl, Cycloheptyl, Cyclooctyl, Cyclopentenyl, Cyclopentadienyl, Cyclohexenyl und Cyclooctadienyl. Ein cyclischer Alkylrest mit 6 C-Atomen ist bevorzugt Cyclohexyl oder Cyclohexenyl.

Die Verbindungen der Formel (I) können durch eine Ringschlussreaktion ausgehend von den entsprechenden β-Ketoestern und anschließender Deacylierung hergestellt werden.
Beispielsweise lassen sich Verbindungen der Formel (la) in einer zweistufigen Reaktion wie in folgendem Schema dargestellt synthetisieren (Nagawade et al. 2005, European Journal of Medicinal Chemistry 40: 1325):

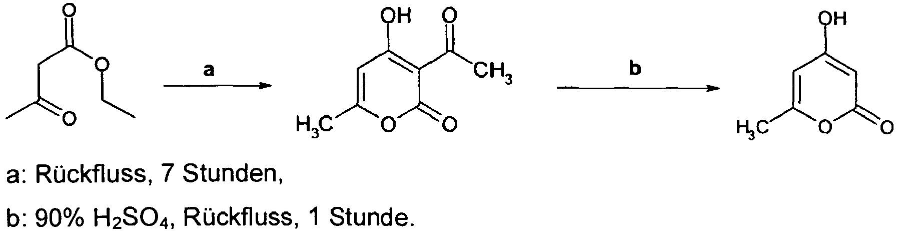

Die entsprechenden Substituenten können dann durch geeignete Alkylierungsreaktionen und anschließende Veretherungs- oder Veresterungsreaktionen eingeführt werden, die dem Fachmann bekannt sind. Ausgehend von Verbindungen der Formel (la) lassen sich beispielsweise durch Veretherung mithilfe von Dimethylsulfat, Bromtetradecan, (E)-1-Brom-3,7-dimethyl-octa-2,6-dien oder Bromheptan Verbindungen der Formel (Ib), (Ic), (Ie) bzw. (Ij) herstellen. Durch Veresterung mit den entsprechenden Säurechloriden können ausgehend von Verbindung (la) beispielsweise die Verbindungen der Formel (Id), (If) oder (Ig) hergestellt werden.

Die Verbindungen der Formel (Ia) (Alfa Aesar) und (Ib) (Aldrich) sowie die weiteren Reaktanden in der Synthese sind kommerziell erhältlich oder durch Synthesen zugänglich, die dem Fachmann aus der Literatur bekannt sind. Dabei bereitet es dem Fachmann keine Schwierigkeiten, die geeigneten Reaktionsbedingungen wie Lösungsmittel oder Temperatur auszuwählen.
Beispiele für mögliche Lösungsmittel sind Acetonitril, Dimethylformamid, Methanol, Pyridin oder Toluol.

Ein weiterer Gegenstand der vorliegenden Erfindung ist eine kosmetische oder pharmazeutische Zubereitung oder ein Nahrungsmittel enthaltend mindestens eine Verbindung der Formel (I) und mindestens einen für topische Anwendungen oder für Nahrungsmittel geeigneten Träger. Bevorzugte Ausführungsformen der Reste R1 bis R6 und X der Formel (I) sind hierbei wie zuvor beschrieben definiert.

Bei den Zubereitungen handelt es sich dabei üblicherweise um topisch anwendbare Zubereitungen, beispielsweise kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe. Bei den Zubereitungen kann es sich auch um Nahrungsmittel handeln. Erfindungsgemäß beinhaltet dieser Begriff auch Nahrungsergänzungsmittel oder "functional food". In diesem Fall enthalten die Zubereitungen einen für Nahrungsmittel geeigneten Träger.

Im Sinne der vorliegenden Erfindung wird neben dem Begriff Zubereitung gleichbedeutend auch der Begriff Mittel oder Formulierung verwendet.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Bevorzugt handelt es sich um eine kosmetische oder pharmazeutische Zubereitung; besonders bevorzugt handelt es sich um eine kosmetische Zubereitung.

Soll die Zubereitung zur Bekämpfung von Cellulite oder zur Reduzierung von lokaler Fettansammlung eingesetzt werden, ist auch eine Zubereitung in Form eines Nahrungsmittels, Nahrungsergänzungsmittels oder "functional food" vorteilhaft. Die orale Verabreichung der Zubereitung führt dabei zur Gewichtsabnahme.

Die mindestens eine Verbindung der Formel (I) wird in den erfindungsgemäßen Zubereitungen typischerweise in Mengen von 0,01 bis 20 Gew.-%, bevorzugt in Mengen von 0,05 bis 10 Gew.-%, besonders bevorzugt in Mengen von 0,1 Gew.-% bis 5 Gew.-% und ganz besonders bevorzugt in Mengen von 0,5 bis 2 Gew.-%, bezogen auf die Gesamtmenge der Zubereitung, eingesetzt. Dabei bereitet es dem Fachmann keinerlei Schwierigkeiten die Mengen abhängig von der beabsichtigten Wirkung der Zubereitung und den gegebenen Bedingungen wie Alter, Gewicht oder Hautzustand entsprechend auszuwählen.

Bei oraler Verabreichung sind typischerweise Mengen von mindestens 0,05 mg/Tag bis 20 mg/Tag nötig. Dabei kann die Dosierung auch entsprechend der gemessenen Veränderung der Cellulite oder des Fettgehalts angepasst werden. Die Dosierung hängt in jedem Fall von Gewicht, Alter und Geschlecht des zu behandelnden Individuums ab.

Die erfindungsgemäßen Zubereitungen können auch neben den Verbindungen der Formel (I) zusätzlich mindestens einen UV-Filter enthalten.

Organische UV-Filter, sogenannte hydrophile oder lipophile Sonnenschutzfilter, sind im UVA-Bereich und/oder UVB-Bereich und/oder IR und/oder VIS-Bereich (Absorber) wirksam. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.

β,β-Diphenylacrylate Derivate: Octocrylene, z. B. vetrieben unter dem Namen "Eusolex® OCR" von der Firma Merck", "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. BASF.

Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine vertrieben als Tinosorb A2B von BASF, 2,2'-[6-(4-methoxyphenyl)-1,3,5-triazine-2,4-diyl]bis[5-(2-ethylhexyl)oxy]-phenol, vertrieben als Tinosorb S von BASF, N2,N4-bis[4-[5-(1,1-dimethylpropyl)-2-benzoxazolyl]phenyl]-N6-(2-ethylhexyl)-1,3,5-triazin-2,4,6-triamin vertrieben als Uvasorb K 2A von der Firma Sigma 3V.

Anthranilin Derivate: Menthyl anthranilate, z. B. vetrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung 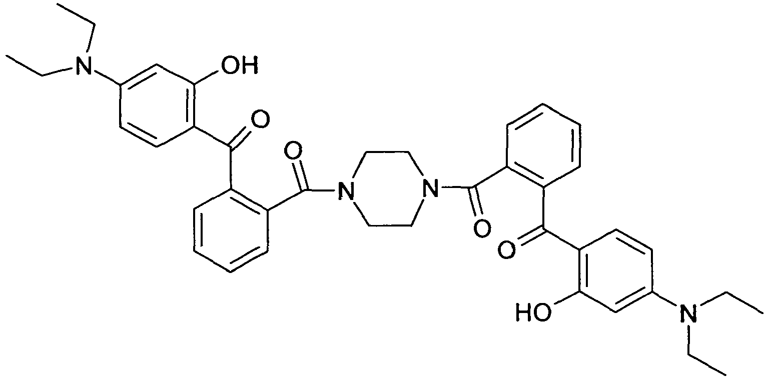 oder die UV-Filter der folgenden Strukturen 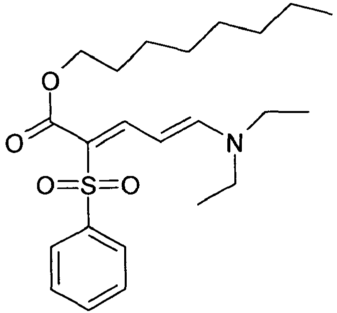 oder

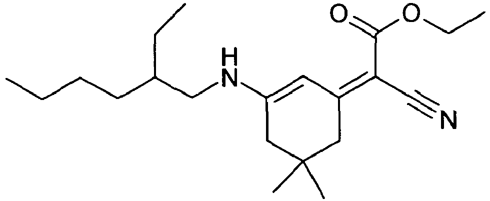

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

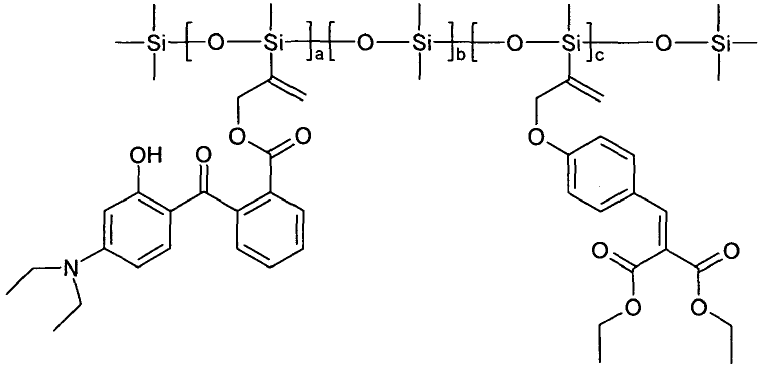

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I sowie den gegebenenfalls organischen UV-Filtern, wie zuvor beschrieben, weitere anorganische UV-Filter, sogenannte partikuläre UV-Filter, enthalten.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53 64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexameta¬phosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   - "Zinc Oxide CS-5" der Fa. Toshibi (ZnO nachbehandelt mit polymethylhydrogenosiloxane)
   - Nanogard Zinc Oxide FN der Fa. Nanophase Technologies
   - "SPD-Z1" der Fa Shin-Etsu (ZnO nachbehandelt mit einem Silikongepfropften Acrylpolymer, dispergiert in Cyclodimethylsiloxane
   - "Escalol Z100" der Fa ISP (Aluminiumoxid nachbehandeltes ZnO dispergiert in einer ethylhexyl methoxycinnamate/PVP-hexadecene/ methicone copolymer Mischung)
   - "Fuji ZNO-SMS-10" der Fa. Fuji Pigment (ZnO nachbehandelt mit Siliciumdioxid und Polymethylsilesquioxan);
   - Unbehandeltes Ceroxide Mikropigment z.B. mit der Bezeichnung "Colloidal Cerium Oxide" der Fa Rhone Poulenc
   - Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandeltem Titandioxid, Zinkoxid-Mischungen wie z.B . das Produkt UV-Titan M261 der Fa. Sachtleben verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

In den beschriebenen Zubereitungen, die erfindungsgemäß mindestens eine Verbindung der Formel I enthalten, können weiterhin auch Farbpigmente enthalten sein, wobei der Schichtaufbau der Pigmente nicht limitiert ist.

Vorzugsweise sollte das Farbpigment bei Einsatz von 0,5 bis 5 Gew.-% hautfarben oder bräunlich sein. Die Auswahl eines entsprechenden Pigments ist für den Fachmann geläufig.

Bevorzugte Zubereitungen können ebenfalls mindestens einen weiteren kosmetischen Wirkstoff enthalten, beispielsweise ausgewählt aus Antioxidantien, Anti-Ageing-, Anti-Falten-, Anti-Schuppen-, Anti-Akne-, Anti-Cellulite-Wirkstoffen, Deodorants, hautaufhellenden Wirkstoffen, Selbstbräunungssubstanzen oder Vitaminen.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA, Pentasodium ethylenediamin tetramethylen phosphonat und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selen¬methionin), Stilbene und deren Derivate (z.B. Stilbenoxid, trans-Stilbenoxid).

Geeignete Antioxidantien sind auch Verbindungen der Formeln A oder B 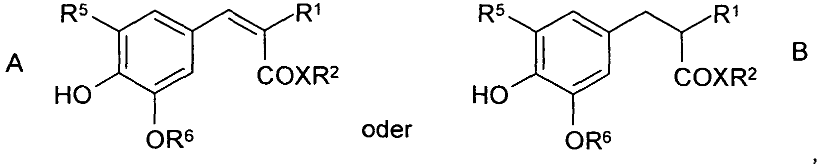 worin
- R¹: aus der Gruppe -C(O)CH₃, -CO₂R³, -C(O)NH₂ und -C(O)N(R⁴)₂ ausgewählt werden kann,
- X: O oder NH,
- R²: lineares oder verzweigtes Alkyl mit 1 bis 30 C-Atomen,
- R³: lineares oder verzweigtes Alkyl mit 1 bis 20 C-Atomen,
- R⁴: jeweils unabhängig voneinander H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen und
- R⁶: lineares oder verzweigtes Alkyl mit 1 bis 8 C-Atomen bedeutet,
vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzyl)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L (+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Die Zubereitungen können auch neben den Verbindungen der Formel (I) ein oder mehrere weitere Anti-Ageing-Wirkstoffe enthalten. Geeignete Anti-Ageing Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin-Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), β-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als Anti-Ageing Wirkstoffe Produkte der Firma Merck wie z.B. 5,7-Dihydroxy-2-methyl-chromon, vermarktet unter dem Handelsnamen RonaCare®Luremine, Ronacare®Isoquercetin, Ronacare®Tilirosid oderRonacare®Cyclopeptide 5 verwendet werden.

Die Zubereitungen können auch ein oder mehrere hautaufhellende Wirkstoffe oder synonym Depigmentierungswirkstoffe oder Melanogeneseinhibitoren enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin, Niacinamide, Azelainsäure, Elaginsäure, Maulbeerbaumextract, Magnesium-ascorbyl-phosphat, Süßholzwurzelextrakt, Emblica, Ascorbinsäure oder Rucinol.

Ferner können die erfindungsgemäßen Zubereitungen mindestens eine Selbstbräunungssubstanz als weiteren Inhaltsstoff enthalten.
Als vorteilhafte Selbstbräunungssubstanzen können unter anderem eingesetzt werden:
1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist 1,3-Dihydroxyaceton, Erythrulose oder deren Kombination. Bevorzugt ist die mindestens eine weitere Selbstbräunungssubstanz in der Zubereitung in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung, enthalten.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die erfindungsgemäßen Zubereitungen können auch ein oder mehrere weitere Anti-Cellulite-Wirkstoffe oder Schlankheitswirkstoffe enthalten. Die zuvor beschriebenen Retinoide eignen sich beispielsweise auch als wirksame Anti-Cellulite-Wirkstoffe. Weitere bekannte Anti-Cellulite-Wirkstoffe sind Phosphodiesterase-Inhibitoren (z.B. Xanthinderivate wie Theophyllin, Koffein, Theobromin oder Salze hiervon wie Aminophyllin) und bestimmte öllösliche Pflanzenextrakte, wie Extrakte von Kletterefeu (Hedera helix), Arnica (Arnica montana), Rosmarin (Rosmarinus officinalis N), Ringelblume (Calendula officinalis), Salbei (Salvia officinalis N), Ginseng (Panax ginseng), Hypericum perforatum, Ruscus aculeatus, Filipendula ulmaria L und Ortosifon stamincus Benth, sowie auch Mischungen dieser Pflanzenextrakte wie sie in US 4,795,638 offenbart werden.
Bevorzugt wird als weiterer Anti-Cellulite-Wirkstoff das Xanthinderivat Koffein oder Theophyllin eingesetzt. Xanthinderivate werden typischerweise in einer Menge von 0,05 Gew.-% bis 20 Gew.-%, bevorzugt in einer Menge von 0,10 Gew.-% bis 10 Gew.-% und besonders bevorzugt in einer Menge von 0,5 Gew.-% bis 3,0 Gew.-% bezogen auf das Gesamtgewicht der Zubereitung, eingesetzt.

Gegenstand der vorliegenden Erfindung ist auch ein Verfahren zur Herstellung einer Zubereitung, wie zuvor beschrieben, dadurch gekennzeichnet, dass mindestens eine Verbindung der Formel (I) mit einem für topische Anwendungen oder für Nahrungsmittel geeigneten Träger und optional mit Hilfs- und oder Füllstoffen vermischt wird. Geeignete Trägerstoffe sowie Hilfs- oder Füllstoffe sind im nachfolgenden Teil ausführlich beschrieben.

Die genannten Bestandteile der Zubereitung können in der üblichen Weise eingearbeitet werden, mit Hilfe von Techniken, die dem Fachmann wohl bekannt sind.

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-ÖI (W/O) oder vom Typ Öl-in-Wasser (O/W), eine multiple Emulsion, beispielsweise vom Typ Waser-in-Öl-in-Wasser (W/O/W) oder O/W/O, ein Gel, einen festen Stift, eine Salbe oder auch ein Aerosol darstellen. Bevorzugt werden Emulsionen. O/W-Emulsinen werden besonders bevorzugt. Emulsionen, W/O-Emulsionen und O/W-Emulsionen sind in üblicher Weise erhältlich.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole, Pflaster, Umschläge, Verbände und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3 Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C Zahl, z.B. mit Isopropanol, Propylenglykol oder Glycerin, oder Ester von Fettalkoholen mit Alkansäuren niedriger C Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C Atomen, aus der Gruppe der Ester aus aromatischen Carbonsäure und gesättigten und/oder ungesättigten, verzweigten und/oder unverzweigten Alkoholen einer Kettenlänge von 3 bis 30 C Atomen. Solche Esteröle können dann vorteilhaft gewählt werden aus der Gruppe Isopropylmyristat, Isopropylpalmitat, Isopropylstearat, Isopropyloleat, n Butylstearat, n-Hexyllaurat, n Decyloleat, Isooctylstearat, Isononylstearat, Isononylisononanoat, 2 Ethylhexylpalmitat, 2 Ethylhexyllaurat, 2 Hexaldecylstearat, 2 Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C Zahl, sowie deren Ether, vorzugsweise Ethanol, Isopropanol, Propylenglykol, Glycerin, Ethylenglykol, Ethylenglykolmonoethyl oder -monobutylether, Propylenglykolmonomethyl, -monoethyl oder -monobutylether, Diethylenglykolmonomethyl oder -monoethylether und analoge Produkte, ferner Alkohole niedriger C Zahl, z. B. Ethanol, Isopropanol, 1,2 Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden. Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate aus folgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann

Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe
Polyethylenglycol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C Atome, Diglycerinester gesättigter und/oder ungesättigter, verzweigter und/oder unverzweigter Alkancarbonsäuren einer Kettenlänge von 8 bis 24, insbesondere 12-18 C¬ Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Damit die Verbindungen der Formel (I) ihre positive Wirkung auf die Haut besonders gut entwickeln können, kann es bevorzugt sein die Verbindungen der Formel (I) in tiefere Hautschichten eindringen zu lassen. Dazu stehen mehrere Möglichkeiten zur Verfügung. Zum einen können die Verbindungen der Formel (I) eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel (I) durch die äußeren Hautschichten ermöglichen. Schließlich ist auch ein systemischer Transport der Verbindungen der Formel (I) denkbar. Die Zubereitung wird dann beispielsweise so gestaltet, dass sie für eine orale Gabe geeignet ist.

Die Verbindungen der Formel (I) können daher erfindungsgemäß auch in Nahrungsmitteln oder als Nahrungsergänzungsmittel oder als "functional food" verwendet werden. Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel (I) angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". Die Nahrungsmittel können fest sein aber auch in flüssiger Form, also als Getränk, vorliegen.

Nahrungsmittel, die nach der vorliegenden Erfindung mit einer oder mehreren Verbindungen der Formel (I) angereichert werden können, sind beispielsweise Nahrungsmittel, die aus einer einzigen natürlichen Quelle stammen (z.B. Zucker, ungesüßter Saft, Korn, Getreide, Getreidesirup), Mischungen von derartigen Nahrungsmitteln (z.B. Multivitaminpräparate, Mineralstoffmischungen oder gezuckerter Saft) oder Nahrungsmittelzubereitungen (z.B. zubereitete Cerealien, Gebäck, Mischgetränke, Joghurt, Diätnahrungsmittel, kalorienarme Nahrungsmittel oder Tierfutter).
Die Nahrungsmittel, die nach der vorliegenden Erfindung mit einer oder mehreren Verbindungen der Formel (I) angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel (I) angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel (I) angereicherten erfindungsgemäßen Nahrungsmittel können mit Hilfe von Techniken hergestellt werden, die dem Fachmann wohl bekannt sind.

Ein weiterer Gegenstand der vorliegenden Erfindung sind auch Verbindungen der Formel (Id) bis (Ij)

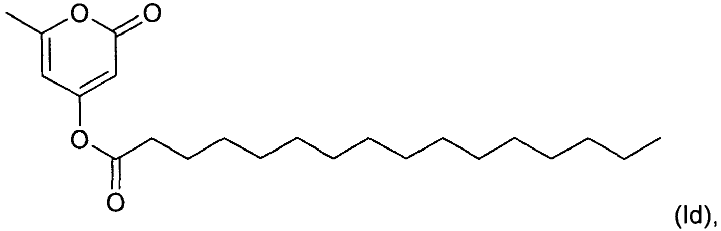

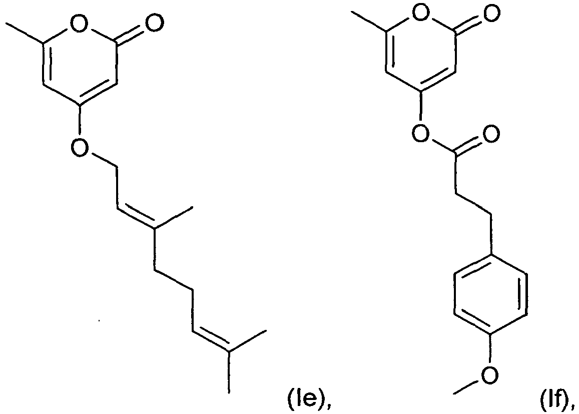

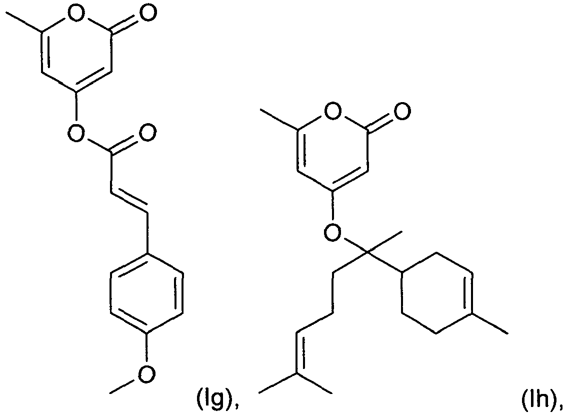

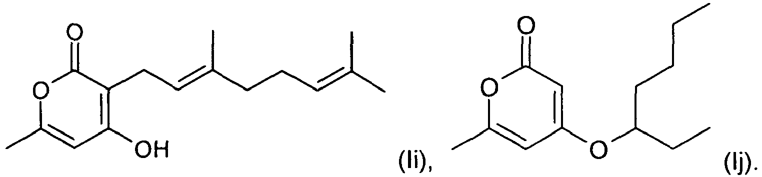

Im folgenden wird die Erfindung anhand von Beispielen näher erläutert. die Erfindung ist im gesamten beanspruchten Bereich ausführbar und nicht auf die hier genannten Beispiele beschränkt.

### Beispiele:

### Beispiel 1: 6-Methyl-4-tetradecyloxy-2-pyron (Ic)

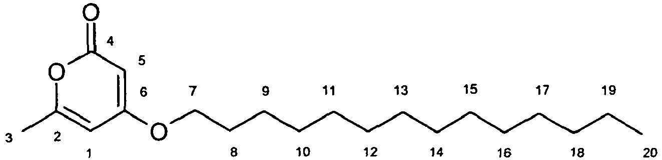

2,50 g (19,8 mmol) 4-Hydroxy-6-methyl-2-pyron (la) und 3,30 mL (23,8 mmol) Triethylamin werden in 12,5 mL Acetonitril vorgelegt. Das Gemisch wird auf 82 °C erhitzt und bei dieser Temperatur wird 6,50 mL (23,8 mmol) 1-Bromtetradecan zugegeben. Die milchig-gelbe Lösung wird nun 16 h unter Rückfluss gekocht. Nach 4 h fängt ein weißer Feststoff an auszufallen. Die Prüfung auf vollständigen Reaktionsumsatz erfolgte durch DC-Analytik (Dichlormethan/Methanol 9:1). Durch Abkühlung der Suspension auf RT fällt so viel Feststoff aus, dass das Gemisch fest wird. Es wird mit Acetonitril verdünnt um es rührbar zu machen, über eine Nutsche abgesaugt, mit Acetonitril nachgewaschen und bei RT im Vakuum getrocknet. Der erhaltene Feststoff wird aus Ethanol umkristallisiert.
Ausbeute: 2,20 g = 34,4 % der Theorie
Farbe: weiß
Summenformel: C₂₀H₃₄O₃
Molmasse: 322,5 g/mol

### Analytik:

MS (EI): m/z (Relative Intensität, %) = 322 ([M+] 14)
¹H-NMR (CDCl₃, 400MHz): δ = 5.78-5.76 (m, 1H, H-1), 5.37 (d, 1H, *J*=2.1 Hz, H-5), 3.92 (t, 2H, *J*=6.5 Hz, H-7), 2.20 (s, 3H, H-3), 1.80-1.71 (m, 2H, H-8), 1.50-1.17 (m, 22H, H-9 bis H-19), 0.88 (t, 3H, *J*=6.7 Hz, H-20)

### Beispiel 2: 4-Hexadecanoyloxy-6-methyl-2-pyron (Id)

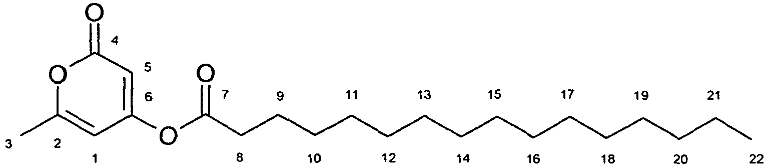

5,00 g (39,6 mmol) 4-Hydroxy-6-methyl-2-pyron (la) und 4,40 g (39,6 mmol) Kalium-tert.-butylat werden in 100 mL N,N-Dimethylformamid vorgelegt. Nun wird 13,2 mL (43,6 mmol) Palmitinsäurechlorid zu der dunkelgelben Suspension bei RT zugetropft und die nun hellgelbe Lösung 16 h bei RT gerührt. Eine Reaktionskontrolle mit DC (Laufmittel: Dichlormethan/Methanol 9:1) zeigt vollständigen Umsatz. Das Reaktionsgemisch wird mit Wasser versetzt, dabei fällt Feststoff aus. Es wird 30 min nachgerührt, dann wird der Feststoff über eine Nutsche abgesaugt, mit Wasser nachgewaschen und im Vakuum bei 40 °C getrocknet. Die Aufreinigung erfolgt durch Kristallisation aus Ethanol. Ausbeute: 4,00 g = 27,7 % der Theorie
Farbe: gelblich
Summenformel: C₂₂H₃₆O₄
Molmasse: 364,5 g/mol

### Analytik:

MS (EI): m/z (Relative Intensität, %) = 364 ([M+] 3)
¹H-NMR (CDCl₃, 400MHz): δ = 6.04-6.02 (m, 1H, H-1), 5.96-5.94 (m, 1H, H-5), 2.52 (t, 2H, *J*=7.5 Hz, H-8), 2.26 (s, 3H, H-3), 1.70 (quint, 2H, *J*=7.4 Hz, H-9), 1.43-1.19 (m, 24H, H-10 bis H-21), 0.88 (t, 3H, *J*=6.7 Hz, H-22).

### Beispiel 3: 4-((E)-3,7-Dimethyl-octa-2,6-dienyloxy)-6-methyl-2-pyron (Ie)

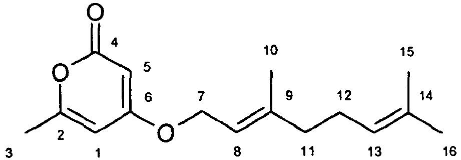

5,00 g (39,6 mmol) 4-Hydroxy-6-methyl-2-pyron und 6,60 g (43,6 mmol) 1,8-Diazabicyclo[5.4.0]undec-7-en (DBU) werden in 80,0 mL Acetonitril vorgelegt und auf 80 °C erhitzt. Dann wird vorsichtig 7,90 mL (39,6 mmol) (E)-1-Brom-3,7-dimethyl-octa-2,6-dien zugegeben und 40 h zum Rückfluss erhitzt. Die Prüfung auf vollständigen Reaktionsumsatz erfolgt durch DC-Analytik (Toluol/Essigester 4:1). Das Reaktionsgemisch wird zum Rückstand eingeengt und mit Toluol/Essigester 9:1 chromatographisch aufgereinigt.
Ausbeute: 1,0 g = 9,6 % der Theorie
Farbe: farblos
Summenformel: C₁₆H₂₂O₃
Molmasse: 262,3 g/mol

### Analytik:

MS (EI): m/z (Relative Intensität, %) = 262 ([M+] 4)
¹H-NMR (CDCl₃, 500MHz): δ = 5.79-5.75 (m, 1H, H-1), 5.41-5.37 (m, 2H, H-5+H-8), 5.12-5.05 (m, 1H, H-13), 4.51 (d, 2H, *J*=6.8 Hz, H-7), 2.19 (s, 3H, H-3), 2.14-2.03 (m, 4H, H-11+H-12), [1.71 (s, 3H), 1.68 (s, 3H), 1.60 (s, 3H)] H-10+H-15+H-16.
¹³C-NMR (DMSO, 300MHz): δ = 170.5 (C-4), 165.2 (C-2 oder C-6), 160.4 (C-2 oder C-6), 143.5 (C-9), 132.1 (C-14), 123.3 (C-13), 117.2 (C-8), 100.7 (C-1), 88.0 (C-5), 65.7 (C-7), 39.5 (C-11), 26.2 (C-12), 25.7 (C-16), 19.8 (C-3), 17.7 (C-15), 16.7 (C-10).

### Beispiel 4: 3-((E)-3,7-Dimethyl-octa-2,6-dienyl)-4-hydroxy-6-methyl-2-pyron (Ii)

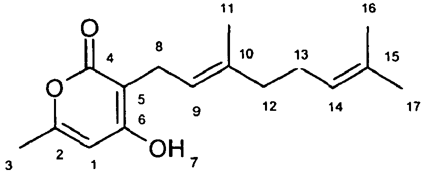

5,00 g (39,6 mmol) 4-Hydroxy-6-methyl-2-pyron und 6,60 g (43,6 mmol) DBU werden in 80,0 mL Acetonitril vorgelegt und auf 80 °C erhitzt. Dann wird vorsichtig 7,90 mL (39,6 mmol) (E)-1-Brom-3,7-dimethyl-octa-2,6-dien zugegeben und 40 h zum Rückfluss erhitzt. Der Umsatz wird mit DC (Laufmittel: Toluol/Essigester 4:1) kontrolliert. Das Reaktionsgemisch wird zum Rückstand eingeengt, mit Toluol/Essigester 4:1, dann mit Essigester/Ethanol 1:1 chromatographisch aufgereinigt.
Ausbeute: 1,60 g = 15,4 % der Theorie
Farbe: gelb
Summenformel: C₁₆H₂₂O₃
Molmasse: 262,3 g/mol

### Analytik:

MS (EI): m/z (Relative Intensität, %) = 262 ([M+] 23)
¹H-NMR (DMSO, 500MHz): δ = 14.49 (s, 1H, H-7), 5.98 (s, 1H, H-1), 5.13-5.07 (m, 1H, H-9), 5.06-5.00 (m, 1H, H-14), 2.95 (d, 2H, *J*=7.1 Hz, H-8), 2.11 (s, 3H, H-3), 1.99 (q, 2H, *J*=7.0 Hz, H-13), 1.89 (t, 2H, *J*=7.0 Hz, H-12), [1.65 (s, 3H), 1.59 (s, 3H), 1.52 (s, 3H)] H-11+H-16+H-17.
¹³C-NMR (DMSO, 300MHz): δ = 164.6 (C-4+C-6), 159.7 (C-2), 134.4 (C-10), 130.6 (C-15), 124.1 (C-14), 121.6 (C-9), 100.6 (C-5), 99.8 (C-1), 38.7 (C-12), 26.1 (C-13), 25.4 (C-17), 21.6 (C-8), 19.2 (C-3), 17.5 (C-16), 15.9 (C-11).

### Beispiel 5: 4-Ethanoyloxy-6-methyl-2-pyron

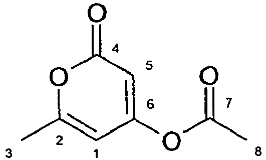

2,00 g (15,9 mmol) 4-Hydroxy-6-methyl-2-pyron und 1,50 mL (15,9 mmol) Essigsäureanhydrid werden in 16,0 mL Toluol suspendiert und dazu werden 16,0 µL (0,30 mmol) Schwefelsäure gegeben. Die Suspension wird 16 h bei RT gerührt, der Umsatz wird mit DC (Laufmittel: Essigester/Heptan 2:1) kontrolliert. Der Feststoff (entspricht dem Ausgangsmaterial) wird abgesaugt, die Mutterlauge wird am Rotationsverdampfer eingeengt. Der erhaltene Rückstand wird über eine Säulenchromatograhie mit Essigester/Heptan 2:3 aufgereinigt.
Ausbeute: 1,20 g = 45,0 % der Theorie
Farbe: gelblich
Summenformel: C₈H₈O₄
Molmasse: 168,1 g/mol

### Analytik:

MS (EI): m/z (Relative Intensität, %) = 168 ([M+] 28)
¹H-NMR (DMSO, 400MHz): δ = 6.30-6.28 (m, 1H, H-1), 6.05-6.02 (m, 1H, H-5), 2.27 (s, 3H, H-3), 2.25-2.23 (m, 3H, H-8).
¹³C-NMR (DMSO, 300MHz): δ = 167.2 (C-4), 163.7 (C-2 oder C-6), 163.5 (C-2 oder C-6), 162.8 (C-7), 101.5 (C-5), 100.6 (C-1), 20.9 (C-8), 19.4 (C-3).

### Beispiel 6: 4-(2-Ethyl-hexanoyloxy)-6-methyl-2-pyron

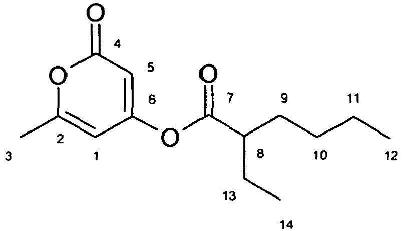

2,50 g (19,8 mmol) 4-Hydroxy-6-methyl-2-pyron und 2,20 g (19,8 mmol) Kalium-tert.-butylat werden in 50,0 mL N,N-Dimethylformamid vorgelegt. Nun wird 3,50 g (21,8 mmol) 2-Ethyl-hexansäurechlorid zu der dunkelgelben Suspension bei RT zugetropft. Das Reaktionsgemisch wird 16 h bei RT gerührt. Eine Reaktionskontrolle mit DC (Laufmittel: Dichlormethan/Methanol 9:1) zeigt vollständigen Umsatz. Das Reaktionsgemisch wird mit Wasser und Tert.-butylmethylether (MtB-Ether) versetzt. Die Phasen werden getrennt, die wässrige Phase wird mit MtB-Ether extrahiert, die vereinten organischen Phasen wurden mit Wasser gewaschen, über Natriumsulfat getrocknet, filtriert und am Rotationsverdampfer eingeengt. Der Rückstand wird über Säulenchromatographie mit Essigester/Heptan 2:1 aufgereinigt.
Ausbeute: 4,10 g = 82,0 % der Theorie
Farbe: gelblich
Summenformel: C₁₄H₂₀O₄
Molmasse: 252,3 g/mol

### Analytik:

MS (EI): m/z (Relative Intensität, %) = 252 ([M+] 7)
¹H-NMR (DMSO, 400MHz): δ = 6.27-6.24 (m, 1H, H-1), 6.03-6.01 (m, 1H H-5), 2.56-2.47 (m, 1H, H-8), 2.26 (s, 3H, H-3), 1.72-1.41 (m, 4H, H-9+H-13), 1.39-1.18 (m, 4H, H-10+H-11), 0.97-0.81 (m, 6H, H-12+H-14) ¹³C-NMR (DMSO, 300MHz): δ = 172.5 (C-4), 163.7+163.3+163.2 (C-2+C-6+C-7), 101.4 (C-1), 101.0 (C-5), 47.4 (C-8), 31.3 (C-9), 29.5 (C-10), 25.2 (C-13), 22.5 (C-11), 20.0 (C-3), 13.8 (C-12), 11.7 (C-14).

### Beispiel 7: 4-(1-Ethyl-pentyloxy)-6-methyl-2-pyron (Ij)

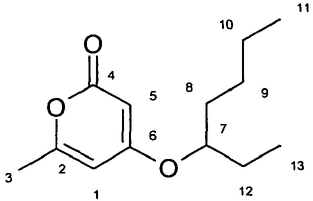

2,50 g (19,8 mmol) 4-Hydroxy-6-methyl-2-pyron und 3,30 mL (23,8 mmol) Triethylamin werden in 12,5 mL Acetonitril vorgelegt. Das Gemisch wird auf 82 °C erhitzt und bei dieser Temperatur wurde 3,90 g (21,8 mmol) 3-Bromheptan zugegeben. Die orange-gelbe Lösung wird nun 16 h unter Rückfluss gekocht. Die Prüfung auf vollständigen Reaktionsumsatz erfolgt durch DC-Analytik (Dichlormethan/Methanol 9:1). Das Reaktionsgemisch wird am Rotationsverdampfer zum Rückstand eingeengt, die anschließende Aufreinigung erfolgt durch Säulenchromatographie mit Essigester/Heptan 2:1.
Ausbeute: 1,40 g = 31,5 % der Theorie
Farbe: gelblich
Summenformel: C₁₃H₂₀O₃
Molmasse: 224,3 g/mol

### Analytik:

MS (EI): m/z (Relative Intensität, %) = 224 ([M+] 15)
¹H-NMR (DMSO, 400MHz): δ = 5.90-5.87 (m, 1H, H-1), 5.52 (d, 1H, *J*=2.2 Hz, H-5), 4.42 (quint, 1H, *J*=5.9 Hz, H-7), 2.16 (s, 3H, H-3), 1.69-1.52 (m, 4H, H-8+H-12), 1.36-1.19 (m, 4H, H-9+H-10), 0.93-0.83 (m, 6H, H-11+H-13).
¹³C-NMR (DMSO, 300MHz): δ = 170.2 (C-4), 165.3 (C-2 oder C-6), 162.0 (C-2 oder C-6), 101.0 (C-1), 87.9 (C-5), 80.3 (C-7), 32.5 (C-8), 27.3 (C-9), 26.1 (C-12), 22.8 (C-10), 19.7 (C-3), 13.9 (C-11), 9.3 (C-13).

### Beispiel 8: cDNA-Array

Der Assay wird mit primären epidermalen Keratinozyten durchgeführt. Nach 48 Stunden Inkubation bei 37 °C und 5%iger CO₂ Atmosphäre wird das Kulturmedium (DPBS und HyQtase-Cell Detachment Solution) entfernt und die Testsubstanzen 4-Hydroxy-6-methyl-2-pyron **Ia** (40 µM) und 3-((E)-3,7-Dimethyl-octa-2,6-dienyl)-4-hydroxy-6-methyl-2-pyron **Ii** (50 µM) in Assay-Medium (Keratinozyten Basal Medium M2 und Supplementmix) zugegeben. All-trans Retinsäure wird als Positivkontrolle verwendet. Nun werden die Zellen bei 37°C und unter 5%igem CO₂ 24 Stunden bebrütet.
Die mRNA wird mit dem Testkit RNEASY Minikit der Firma Qiagen nach Anleitung isoliert. Nun wird jeweils 1 µg der RNA mit Hilfe des Firsts Strand cDNA Synthese Kits der Firma Roche in cDNA umgeschrieben. Die erhaltenen 20 µL cDNA werden 1/10 verdünnt und 110 µL der cDNA-Proben werden jeweils mit 110 µL TaqMan™ Universal PCR Master Mix versetzt. 100 µL werden pro Slot in die Custom TaqMan™ Array Karten pipettiert. Die Karten werden verschlossen, zentrifugiert und dann mit dem TaqMan™ 7900HT vermessen. Die erhaltenen Werte werden normalisiert. Werte kleiner oder gleich -1,5 weisen auf eine mittlere bis starke Herunterregulierung der Expression der untersuchten Gene hin.

Die Einflüsse von Hydroxy-6-methyl-2-pyron **Ia** (40 µM) und 3-((E)-3,7-Dimethyl-octa-2,6-dienyl)-4-hydroxy-6-methyl-2-pyron **Ii** (50µM) auf die Genexpression der Zellhüllproteine Calmodulin-like Protein (CALML5), Filaggrin (FLG), Involukrin (IVL), Lorikrin (LOR), Repetin (RPTN), Calgranulin A und B (S100A8, S100A9) und Transglutaminase 1 (TGM1) sind in Tabelle 1 dargestellt.

**Tabelle 1**

| Applikation | CALML5 | FLG | IVL | LOR |
|---|---|---|---|---|
| all-trans Retinsäure (5µM) | -2,93 | -3,20 | -4,05 | -7,01 |
| **Ia** (40 µM) | -3,14 | 1,01 | -1,76 | -4,56 |
| **Ii** (50 µM) | -2,58 | -4,67 | -3,82 | -1,59 |
| | | | | |

| | RPTN | S100A8 | S100A9 | TGM1 |
|---|---|---|---|---|
| all-trans Retinsäure (5µM) | -2,56 | -3,26 | -3,49 | -3,56 |
| **Ia**(40 µM) | -3,33 | -1,74 | -2,18 | -1,26 |
| **Ii** (50 µM) | 1,57 | -2,93 | -1,48 | -1,56 |

Für Hydroxy-6-methyl-2-pyron **Ia** (40 µM) zeigt die Tabelle bis auf Filaggrin eine hemmende Wirkung der mRNA-Produktion für das "cornified envelope". Auch Verbindung **Ii** zeigt bis auf Repetin, einheitliche Herunterregulierung der Genexpression. Der Vergleich all-trans Retinsäure zeigt ein homogenes Bild der Reduktion von mRNA für alle Zellhüllproteine. Die Herunterregulierung dieser Genfamilie ist ein typisches Merkmal von Substanzen, die eine anti-differenzierende Wirkung aufweisen.

Eine zweite Gruppe von Genen (Tabelle 2), die betrachtet wird, kodiert für Proteine, die mit den Zell-Zell-Verbindungen zusammenhängen. Getestet werden Corneodesmosin (CDSN), Desmoglein 1 (DSG1), Desmoplakin (DSP) und Desmocollin 3 (DSC3), die zugehörigen Regulationsfaktoren sind in Tabelle 2 dargestellt. Die Expression aller Gene wird durch **Ia** und **Ii** herunter reguliert. Die Herunterregulierung dieser Proteinfamilie ist für Retinoide bekannt.

**Tabelle 2.**

| Applikation | CDSN | DSC3 | DSG1 | DSP |
|---|---|---|---|---|
| all-trans Retinsäure (µM) | -2,37 | -1,23 | -9,22 | -1,56 |
| 4-Hydroxy-6-methyl-2-pyron **Ia** (40 µM) | -2,14 | -1,69 | -1,39 | -1,40 |
| 3-(((E)-3,7-Dimethyl-octa-2,6-dienyl)-4-hydroxy-6-methyl-2-pyron **Ii** (50 µM) | -13,20 | -1,14 | -8,20 | -1,53 |

Die Ergebnisse des cDNA-Arrays zeigen ein ähnliches Expressionsmuster wie Retinsäure.

### Beispiel 9: Ex-vivo Studie

Aus dem Unterleibsgewebe einer 45 jährigen europäischen Frau werden histologische Explante mit einem mittleren Durchmesser von 10 mm vorbereitet. Die Gewebestücke werden in BEM Medium in einer feuchten, 5%igen CO₂ Atmosphäre bei 37 °C gelagert. Eingeteilt wird das Gewebe in 3 Chargen von je 6 Explanten.

**Testsubstanzen und Probennahme**

| | Applikation | Probennahme |
|---|---|---|
| K | - | Tag 8 |
| V | Mygliol/Ethanol (80/20) | Tag 8 |
| P | 4-Hydroxy-6-methyl-2-pyron **Ia** (1% in Mygliol/Ethanol 80/20) | Tag 8 |

Charge K stellt die Negativkontrolle dar. Auf die Charge V wird nur das Vehikel aufgegeben. Die Charge P wird mit 4-Hydroxy-6-methyl-2-pyron **Ia** (1% in Mygliol/Ethanol 80/20) behandelt. Es werden 30 µL der 1%igen Substanz-Lösungen in Myliol/Ethanol (80/20) unter Verwendung eines runden Filterpapiers appliziert und 2 Stunden einwirken gelassen. Applikationen finden am Tag 0, 1, 4, 6 und 7 statt.

Am 8. Tag werden je 3 Gewebestücke von jeder Charge in Bouin-Lösung fixiert. Nach der 48 stündigen Fixierung werden die Proben mittels Leica TP 1020 dehydriert und in Paraffin getränkt. Aus den Paraffinblöcken werden mit einem Mikrotom 5 µm dicke Gewebeschnitte hergestellt und auf Superfrost Objektträger aufgezogen. Die mikroskopische Untersuchung wird mit einem Leica Orthoplan Mikroskop durchgeführt, nachdem die auf Objektträger aufgezogenen Gewebeschnitte mit Masson-Goldner-Trichromfärbung angefärbt worden sind. Um die Wirkung der Substanzen beurteilen zu können, wird die Dicke der Epidermis bzw. deren Änderung gemessen.

Ergebnisse: Am Tag 0 werden die eingesetzten Haut-Explante morphologisch untersucht und dann das erste Mal die Testsubstanzen topisch appliziert. Die allgemeine Morphologie der Hautschnitte zeigt ein mäßig dickes Stratum corneum.

Nach 8 Tagen ist die Morphologie der unbehandelten Haut (Charge K) fast unverändert.
Das Hautgewebe, das dem Vehikel ausgesetzt wird (Charge V), zeigt nur eine geringe Veränderung des Stratum granulosum und eine Verringerung der Parakeratose, die in unbehandelter Haut vorhanden ist.
Die Behandlung mit Substanz **Ia** (Charge P) induziert ein mäßiges Wachstum der Epidermis. Im Vergleich zum Vehikel wird nach Einwirkung mit Verbindung **Ia** am 8. Tag eine 10 % dickere Epidermis gemessen. Das Kollagennetzwerk in der Dermis entlang der dermo-epidermalen Junktionszone wird dichter.

### Beispiel 10: Lipolyse

Die Studie wird mit normalen humanen Adipozyten, die aus der Unterleibsbiopsie einer 31 jährigen Frau stammen, durchgeführt. Aus der Biopsie wird die Hypodermis isoliert und 30 Minuten mit Collagenase-Lösung bei 37°C inkubiert. Die getrennten Adipozyten werden gewaschen und mit Assay-Medium (MEM ohne Phenolrot, Penicillin 25 UI/mL/Streptomycin 25 µL/mL, L-Glutamin 2mM, Rinderserum Albumin (BSA), fettsäurefrei: 0,5 % (p/v)) verdünnt.

Die Testlösungen oder die Referenz Coffein werden zu den Zellsuspensionen gegeben und diese werden bei 37°C und 5%iger CO₂ Atmosphäre 2 Stunden inkubiert. Die getesteten Pyrone und Konzentrationen sind in der Tabelle aufgeführt.

Nach dieser Zeit werden von den Proben die freigesetzten unveresterten Fettsäuren quantifiziert. Die Messung wird mit einem NEFA-C Test-Kit der Firma Wako nach Anleitung durchgeführt.

**Testsubstanzen und -konzentrationen für die Lipolyse**

| **Testsubstanz** | **Testkonzentrationen** |
|---|---|
| Coffein | 1 mM |
| 4-Methoxy-6-methyl-2-pyron **Ib** | 0,25 mM; 0,5 mM;1 mM und 2 mM |
| 3-((E)-3,7-Dimethyl-octa-2,6-dienyl)-4-hydroxy-6-methyl-2-pyron **Ii** | 0,25 mM; 0,5 mM; 1 mM und 2 mM |
| 4-(1-Ethyl-pentyloxy)-6-methyl-2-pyron **Ij** | 0,25 mM; 0,5 mM; 1 mM und 2 mM |

Ergebnisse: Verbindung **Ib** fördert den Triacylglycerin-Abbau (82% Stimulierung signifikant bei 2mM). Verbindung **Ij** zeigt einen ähnlichen Verlauf (57% Stimulierung signifikant bei 2mM). Beide Substanzen erhöhen die lipolytische Aktivität konzentrationsabhängig.

Die Ergebnisse für Verbindung **Ii** (-67 % (bei 2mM) bzw. -60 % (bei 1mM)) weisen darauf hin, dass Verbindung Ii dem bei der Hautalterung einsetzenden Abbau des subkutanen Fettgewebes entgegenwirken kann (Füllungs-Effekt für Anti-Aging).

### Beispiel 11: O/W-Formulierung

| **Bestandteile /Handelsname** | **Bezugsquelle** | **INCI** | **[Gew-%]** |
|---|---|---|---|
| **A** | | | |
| Marlipal 1618/11 | (1) | CETEARETH-11 | 3 |
| Lanette O | (2) | CETEARYLALCOHOL | 7 |
| Luvitol EHO | (3) | CETEARYLOCTANOATE | 5 |
| Tegosoft TN | (4) | C12-15 ALKYLBENZOATE | 2.5 |
| Miglyol 812 N | (1) | CAPRYLIC/CAPRIC TRIGLYCERIDE | 2.5 |
| Propyl-4-hydroxybenzoat | (5) | PROPYLPARABEN | 0.05 |
| 3-((E)-3,7-Dimethyl-octa-2,6-dienyl)-4-hydroxy-6-methyl-2-pyron | | | 0.05 |
| | | | |

| **B** | | | |
|---|---|---|---|
| 1,2-Propandiol | (5) | PROPYLENE GLYCOL | 4 |
| Methyl-4-hydroxybenzoat | (5) | METHYLPARABEN | 0.15 |
| Wasser, demineralisiert | | AQUA (WATER) | ad 100 |
| | | | |
| Wasser, demineralisiert | | | 10 |
| **Gesamt** | | | **100.00** |

### Herstellungsverfahren:

Zunächst wird die Phase A auf 75°C und die Phase B auf 80 °C erwärmt. Danach wird Phase B unter Rühren langsam zu Phase A gegeben und solange gerührt, bis eine homogene Mischung entsteht.

### Bezugsguellen:

(1) Sasol Germany GmbH (2) Cognis GmbH (3) BASF AG (4) Degussa-Goldschmidt AG (5) Merck KGaA/Rona®

### Beispiel 12: O/W-Formulierung

| **Bestandteile /Handelsname** | **Bezugsquelle** | **INCI** | **[Gew-%]** |
|---|---|---|---|
| **A** | | | |
| Marlipal 1618/11 | (1) | CETEARETH-11 | 3 |
| Lanette O | (2) | CETEARYLALCOHOL | 7 |
| Luvitol EHO | (3) | CETEARYLOCTANOATE | 5 |
| Tegosoft TN | (4) | C12-15 ALKYLBENZOATE | 2.5 |
| Miglyol 812 N | (1) | CAPRYLIC/CAPRIC TRIGLYCERIDE | 2.5 |
| Propyl-4-hydroxybenzoat | (5) | PROPYLPARABEN | 0.05 |
| Retinol | (3) | (2E,4E,6E,8E)-3,7-Dimethyl- 9-(2,6,6-trimethylcyclohex-1 - enyl)nona- 2,4,6,8-tetrae 1-ol | 0.04 |
| 3-((E)-3,7-Dimethyl-octa-2,6-dienyl)-4-hydroxy-6-methyl-2-pyron (Ii) | | | 0.1 |
| | | | |

| **B** | | | |
|---|---|---|---|
| 1,2-Propandiol | (5) | PROPYLENE GLYCOL | 4 |
| Methyl-4-hydroxybenzoat | (5) | METHYLPARABEN | 0.15 |
| Wasser, demineralisiert | | AQUA (WATER) | ad 100 |
| | | | |
| Wasser, demineralisiert | | | 10 |
| **Gesamt** | | | **100.00** |

### Herstellungsverfahren:

Zunächst wird die Phase A auf 75°C und die Phase B auf 80 °C erwärmt. Danach wird Phase B unter Rühren langsam zu Phase A gegeben und solange gerührt, bis eine homogene Mischung entsteht.

### Bezugsguellen:

(1) Sasol Germany GmbH (2) Cognis GmbH (3) BASF AG (4) Degussa-Goldschmidt AG (5) Merck KGaA/Rona®

### Beispiel 13: O/W-Formulierung

| **Bestandteile / Handelsname** | **Bezugsquelle** | **INCI** | **[Gew-%]** |
|---|---|---|---|
| **A** | | | |
| Tego Care 150 | (1) | GLYCERYL STEARATE, STEARETH-25, CETETH-20, STEARYL ALCOHOL | 8 |
| Lanette O | (2) | CETEARYL ALCOHOL | 1.5 |
| Luvitol EHO | (3) | CETEARYL OCTANOATE | 5 |
| Miglyol 812 N | (4) | CAPRYLIC/CAPRIC TRIGLYCERIDE | 5 |
| Paraffin liquid | (5) | PARAFFINUM | 3 |
| | | LIQUIDUM (MINERAL OIL) | |
| AbilWax 2434 | (1) | STEAROXY DIMETHICONE | 1.6 |
| Dow Corning 200 Fluid (350 cs) | (6) | DIMETHICONE | 0.5 |
| Propyl-4-hydroxybenzoate | (5) | PROPYLLPARABEN | 0.05 |
| | | | |

| **B** | | | |
|---|---|---|---|
| 1,2-Propanediol | (5) | PROPYLENE GLYCOL | 3 |
| Methyl-4-hydroxybenzoate Water, demineralized | (5) | METHYLPARABEN AQUA (WATER) | 0.15 ad 100 |

| C | | | |
|---|---|---|---|
| | | | |
| Probiol L 05018 (Empty liposomes) | (7) | AQUA, ALCOHOL DENAT, LECITHIN, GLYCERINE, DISODIUM | 5 |
| | | PHOSPHATE | |
| Water, demineralized | | AQUA (WATER) | 10.00 |
| 4-Hydroxy-6-methyl-2-pyron | | | 1 |
| | | | |
| **Gesamt** | | | **100.00** |

### Herstellungsverfahren:

Zunächst werden die Phasen A und B auf 80 °C erwärmt. Danach wird Phase B unter Rühren langsam zu Phase A gegeben und homogenisiert. Dann wird abgekühlt und die Phase C bei 40°C zugegeben.

### Bezugsquellen:

(1) Degussa-Goldschmidt AG, (2) Cognis GmbH, (3) BASF AG, (4) Sasol Germany GmbH, (5) Merck KGaA/Rona®, (6) Dow Corning, (7) Kuhs GmbH & Co. KG

### Beispiel 14: W/O-Formulierung

| **Bestandteile / Handelsname** | **Bezugsquelle** | **INCI** | **[Gew-%]** |
|---|---|---|---|
| **A** | | | |
| Dow Corning 3225 C | (1) | CYCLOMETHICONE, DIMETHICONE COPOLYOL | 23.6 |
| Propyl-4-hydroxybenzoate | (2) | PROPYLPARABEN | 0.05 |
| 4-Hydroxy-6-methyl-2-pyron | | | 1 |
| | | | |

| **B** | | | |
|---|---|---|---|
| Methyl-4-hydroxybenzoate | (2) | METHYLPARABEN | 0.15 |
| 1,2-Propanediol | (2) | PROPYLENE GLYCOL | 35.9 |
| Water, demineralized | | AQUA (WATER) | ad 100 |
| | | | |
| **Gesamt** | | | **100.00** |

### Herstellungsverfahren:

Zunächst wird die Phase B aufgelöst und dann wird sie zu Phase A gegeben. Der pH-Wert wird mit Natronlauge bzw. Citronensäure auf den Wert pH = 6.0 eingestellt.

### Bezugsguellen:

(1) Dow Corning (2) Merck KGaA/Rona®

### Beispiel 15: O/W Anti-aging Creme mit UV A/B Schutz

| **Bestandteile / Handelsname** | **Bezugsquelle** | **INCI** | **[Gew-%]** |
|---|---|---|---|
| **A** | | | |
| Eusolex® 2292 | (1) | ETHYLHEXYL METHOXYCINNAMATE, BHT | 3 |
| Eusolex® 4360 | (1) | BENZOPHENONE-3 | 0.5 |
| Tego Care 150 | (2) | GLYCERYL STEARATE, STEARETH-25, CETETH-20, STEARYL ALCOHOL | 8 |
| Lanette O | (3) | CETEARYL ALCOHOL | 1.5 |
| Luvitol EHO | (4) | CETEARYL OCTANOATE | 5 |
| Miglyol 812 N | (5) | CAPRYLIC/CAPRIC TRIGLYCERIDE | 5 |
| Paraffin liquid | (1) | PARAFFINUM LIQUIDUM (MINERAL OIL) | 3 |
| Abil-Wax 2434 | (2) | STEAROXY DIMETHICONE | 1.6 |
| Dow Corning 200 Fluid (350 | (6) | DIMETHICONE | 0.5 |
| cs) | | | |
| Propyl-4-hydroxybenzoate | (1) | PROPYLPARABEN | 0.05 |
| | | | 1 |
| 3-((E)-3,7-Dimethyl-octa-2,6-dienyl)-4-hydroxy-6-methyl-2-pyron (Ii) | | | |
| | | | |

| **B** | | | |
|---|---|---|---|
| 1,2-Propanediol | (1) | PROPYLENE GLYCOL | 3 |
| Methyl-4-hydroxybenzoate sodium salt | (1) | SODIUM METHYLPARABEN | 0.17 |
| Wasser, demineralisiert | | AQUA (WATER) | ad 100 |
| | | | |
| **Gesamt** | | | **100.00** |

### Herstellungsverfahren:

Zunächst werden die Phasen A und B getrennt gemischt und auf 80°C erwärmt. Danach wird Phase B langsam unter Rühren zu Phase A gegeben. Es wird homogenisiert auf Raumtemperatur abgekühlt.

### Bezugsquellen:

(1) Merck KGaA/Rona®, (2) Degussa-Goldschmidt AG, (3) Cognis GmbH, (4) BASF AG, (5) Sasol Germany GmbH, (6)

### Beispiel 16: Anti-cellulite-Creme

| Bestandteile | % |
|---|---|
| **A** | |
| Cetyl alcohol | 2 |
| Glyceryl Stearate | 5 |
| Caprylic/Capric Triglyceride | 8 |
| Isopropyl Palmitate | 9 |
| 4-methoxy-6-methyl-2-pyrone | 1 |
| | |

| **B** | |
|---|---|
| Glycerin | 3 |
| Preservatives (Germaben II) | 0.8 |
| Water, entmineralisiert | ad 100 |

### Herstellungsverfahren:

Zunächst wird die Phase B aufgelöst und dann wird sie zu Phase A gegeben. Der pH-Wert wird mit Natronlauge bzw. Citronensäure auf den Wert pH = 6.0 eingestellt.

### Anwendung:

Zweimal täglich unter kräftigem Massieren auf die Haut auftragen; dabei kreisende und Auf- und Abbewegungen ausführen. Die Behandlung kann sowohl auf Oberschenkeln, als auch Gesäß und Bauch durchgeführt werden.

## Patentansprüche

1. Nicht-therapeutische Verwendung mindestens einer Verbindung der Formel (I) 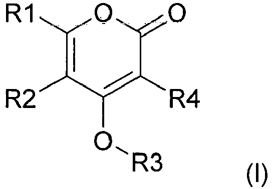 wobei
R1 für eine geradkettige oder verzweigte C₁- bis C₂₀-Alkylgruppe steht,
R2 steht für
- H,
R4 steht für
- H,
- geradkettige oder verzweigte C₁- bis C₂₀-Alkylgruppe oder
- geradkettige oder verzweigte C₂- bis C₂₀-Alkenylgruppe mit einer oder mehreren Doppelbindungen,
R3 steht für einen Rest ausgewählt aus
- H,
- geradkettige oder verzweigte C₁- bis C₂₀-Alkylgruppe,
- geradkettige oder verzweigte C₂- bis C₂₀-Alkenylgruppe mit einer oder mehreren Doppelbindungen, wobei die Alkenylgruppe auch mit einer oder mehreren gesättigten oder ungesättigten C₃- bis C₁₂-Cycloalkylgruppen substituiert sein kann,
- geradkettige oder verzweigte C₂- bis C₂₀-Alkinylgruppe mit einer oder mehreren Dreifachbindungen,
- gesättigte oder ungesättigte C₃- bis C₁₂-Cycloalkylgruppe, wobei die Ringe jeweils auch durch -(CH₂)ₙ-Gruppen mit n = 1 bis 3 überbrückt sein können,
- ein Acylrest der Formel -C(=0)-R6,
R6 steht für
- geradkettige oder verzweigte C₁- bis C₂₀-Alkylgruppe,
- einen Rest der Formel (II) 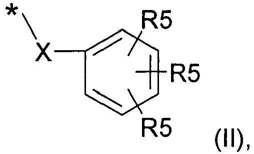 worin X für geradkettiges oder verzweigtes C₁- bis C₆-Alkylen oder geradkettiges oder verzweigtes C₂- bis C₆-Alkenylen steht und die Reste R5 unabhängig voneinander ausgewählt sind aus H, OH, geradkettiges oder verzweigtes C₁- bis C₆-Alkyl oder geradkettiges oder verzweigtes O-(C₁- bis C₆-Alkyl),
zur Pflege, Konservierung oder Verbesserung des allgemeinen Zustands oder des Erscheinungsbildes der Haut oder Haare.

2. Verwendung nach Anspruch 1 zur Vorbeugung, Reduzierung oder Bekämpfung von Cellulite oder von Anzeichen von Cellulite und/oder zur Reduzierung von lokaler Fettansammlung.

3. Verwendung nach Anspruch 1 zur Prophylaxe gegen oder Bekämpfung von zeit- und/oder lichtinduzierten Alterungsprozessen der Haut oder Haare.

4. Verwendung nach Anspruch 1 oder 3 zur Prophylaxe gegen oder Reduktion von Hautunebenheiten, wie Falten, feinen Linien, rauher Haut oder großporiger Haut.

5. Verwendung nach Anspruch 2 zur Stimulierung der Lipolyse.

6. Verwendung nach einem oder mehreren der Ansprüche 1 bis 5, **dadurch gekennzeichnet, dass** R1 für eine geradkettige oder verzweigte C₁- bis C₆-Alkylgruppe steht.

7. Verwendung nach einem oder mehreren der Ansprüche 1 bis 6, **dadurch gekennzeichnet, dass** R4 für H oder eine geradkettige oder verzweigte C₂- bis C₂₀-Alkenylgruppe mit einer oder mehreren Doppelbindungen steht.

8. Verwendung nach einem oder mehreren der Ansprüche 1 bis 7, **dadurch gekennzeichnet, dass** R3 für einen Rest steht ausgewählt aus
- H,
- geradkettige oder verzweigte C₁- bis C₂₀-Alkylgruppe,
- geradkettige oder verzweigte C₂- bis C₂₀-Alkenylgruppe mit einer oder mehreren Doppelbindungen, wobei die Alkenylgruppe auch mit einer oder mehreren gesättigten oder ungesättigten Cyclohexylgruppen substituiert sein kann,
- ein Acylrest der Formel -C(=O)-R6.

9. Verwendung nach einem oder mehreren der Ansprüche 1 bis 8, **dadurch gekennzeichnet, dass** die Reste R5 unabhängig voneinander ausgewählt sind aus H, OH oder geradkettiges oder verzweigtes O-(C₁-bis C₆-Alkyl).

10. Verwendung nach einem oder mehreren der Ansprüche 1 bis 9, **dadurch gekennzeichnet, dass** die Verbindung der Formel (I) ausgewählt ist aus den Verbindungen der Formel (Ia) bis (Ij)

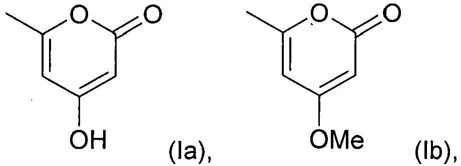

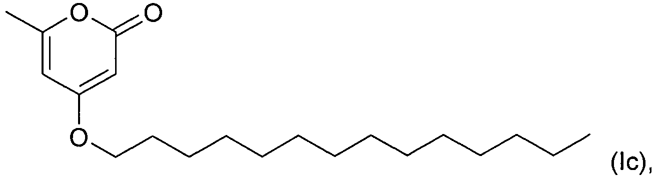

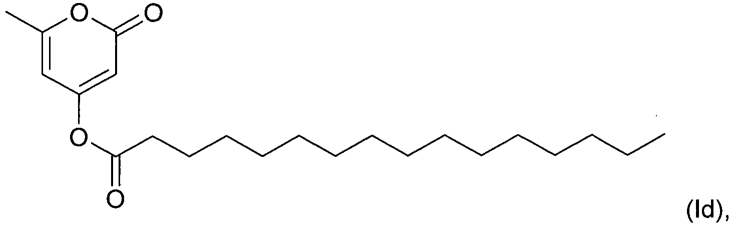

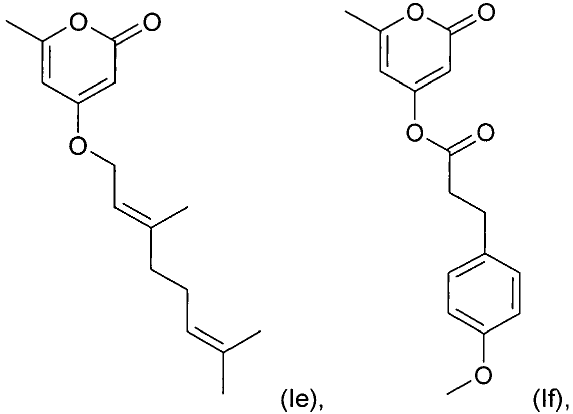

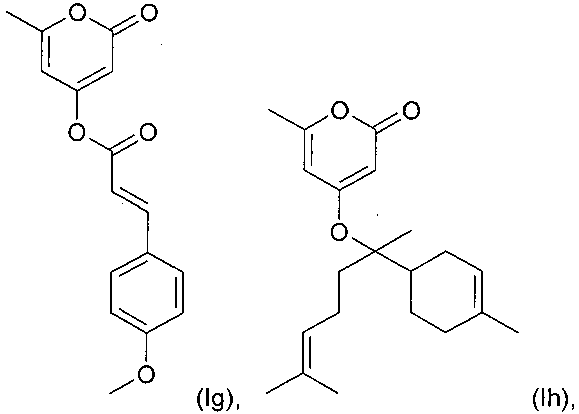

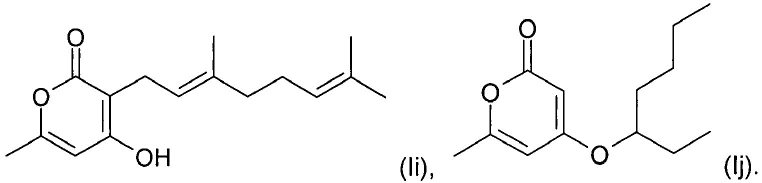

11. Kosmetische oder pharmazeutische Zubereitung oder ein Nahrungsmittel enthaltend mindestens eine Verbindung der Formel (I) 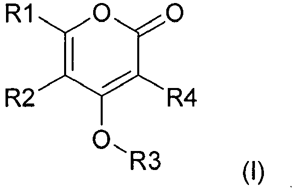 wobei
R1 für eine geradkettige oder verzweigte C₁- bis C₂₀-Alkylgruppe steht,
R2 steht für
- H,
R4 steht für
- H,
- geradkettige oder verzweigte C₁- bis C₂₀-Alkylgruppe oder
- geradkettige oder verzweigte C₂- bis C₂₀-Alkenylgruppe mit einer oder mehreren Doppelbindungen,
R3 steht für einen Rest ausgewählt aus
- H,
- geradkettige oder verzweigte C₁- bis C₂₀-Alkylgruppe,
- geradkettige oder verzweigte C₂- bis C₂₀-Alkenylgruppe mit einer oder mehreren Doppelbindungen, wobei die Alkenylgruppe auch mit einer oder mehreren gesättigten oder ungesättigten C₃- bis C₁₂-Cycloalkylgruppen substituiert sein kann,
- geradkettige oder verzweigte C₂- bis C₂₀-Alkinylgruppe mit einer oder mehreren Dreifachbindungen,
- gesättigte oder ungesättigte C₃- bis C₁₂-Cycloalkylgruppe, wobei die Ringe jeweils auch durch -(CH₂)ₙ-Gruppen mit n = 1 bis 3 überbrückt sein können,
- ein Acylrest der Formel -C(=O)-R6,
R6 steht für
- geradkettige oder verzweigte C₁- bis C₂₀-Alkylgruppe,
- einen Rest der Formel (II) 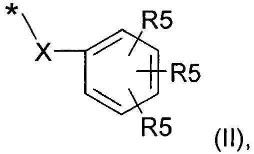 worin X für geradkettiges oder verzweigtes C₁- bis C₆-Alkylen oder geradkettiges oder verzweigtes C₂- bis C₆-Alkenylen steht und die Reste R5 unabhängig voneinander ausgewählt sind aus H, OH, geradkettiges oder verzweigtes C₁- bis C₆-Alkyl oder geradkettiges oder verzweigtes O-(C₁- bis C₆-Alkyl),
und mindestens einen für topische Anwendungen oder für Nahrungsmittel geeigneten Träger.

12. Zubereitung nach Anspruch 11, **dadurch gekennzeichnet, dass** die mindestens eine Verbindung der Formel (I) in einer Menge von 0,01 bis 20 Gew.-% enthalten ist.

13. Verfahren zur Herstellung einer Zubereitung nach Anspruch 11 oder 12, **dadurch gekennzeichnet, dass** die Verbindung der Formel (I) mit einem für topische Anwendungen oder für Nahrungsmittel geeigneten Träger vermischt wird.

14. Verbindungen der Formel (Id) bis (Ij)

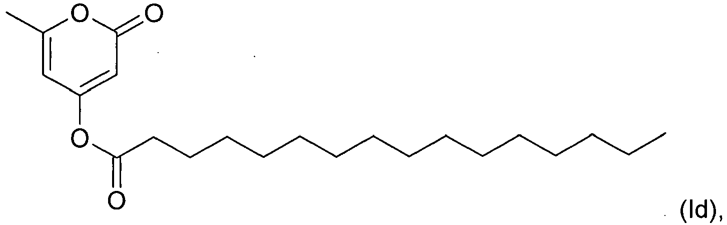

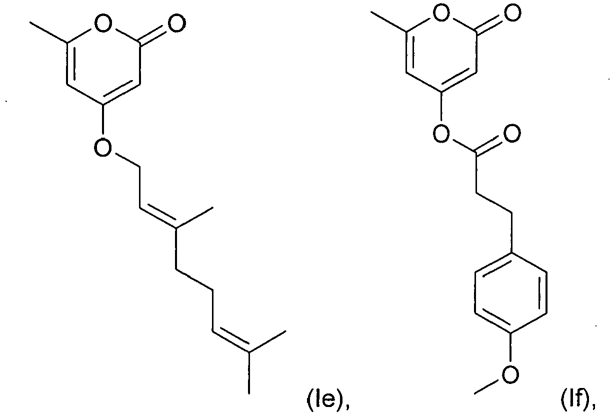

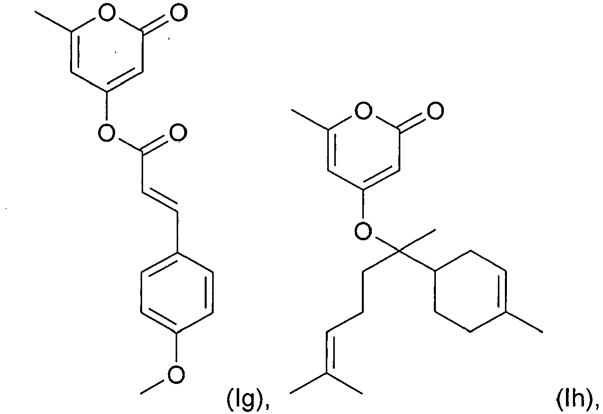

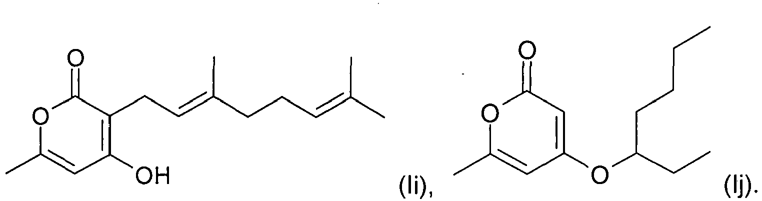

## Claims

1. Non-therapeutic use of at least one compound of the formula (I) 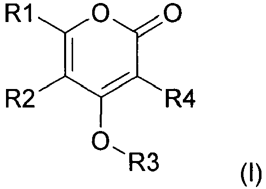 where
R1 stands for a straight-chain or branched C₁- to C₂₀-alkyl group,
R2 stands for
- H,
R4 stands for
- H,
- straight-chain or branched C₁- to C₂₀-alkyl group or
- straight-chain or branched C₂- to C₂₀-alkenyl group having one or more double bonds,
R3 stands for a radical selected from
- H,
- straight-chain or branched C₁- to C₂₀-alkyl group,
- straight-chain or branched C₂- to C₂₀-alkenyl group having one or more double bonds, where the alkenyl group may also be substituted by one or more saturated or unsaturated C₃- to C₁₂-cycloalkyl groups,
- straight-chain or branched C₂- to C₂₀-alkynyl group having one or more triple bonds,
- saturated or unsaturated C₃- to C₁₂-cycloalkyl group, where the rings may in each case also be bridged by -(CH₂)ₙ- groups where n = 1 to 3,
- an acyl radical of the formula -C(=O)-R6,
R6 stands for
- straight-chain or branched C₁- to C₂₀-alkyl group,
- a radical of the formula (II) 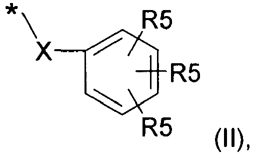 in which X stands for straight-chain or branched C₁- to C₆alkylene or straight-chain or branched C₂- to C₆-alkenylene and the radicals R5 are selected, independently of one another, from H, OH, straight-chain or branched C₁- to C₆alkyl or straight-chain or branched O-(C₁- to C₆-alkyl),
for the care, preservation or improvement of the general condition or appearance of the skin or hair.

2. Use according to Claim 1 for the prevention, reduction or combating of cellulite or signs of cellulite and/or for the reduction of local fat accumulation.

3. Use according to Claim 1 for prophylaxis against or combating of time-and/or light-induced ageing processes of the skin or hair.

4. Use according to Claim 1 or 3 for prophylaxis against or reduction of skin unevenness, such as wrinkles, fine lines, rough skin or large-pored skin.

5. Use according to Claim 2 for the stimulation of lipolysis.

6. Use according to one or more of Claims 1 to 5, **characterised in that** R1 stands for a straight-chain or branched C₁- to C₆-alkyl group.

7. Use according to one or more of Claims 1 to 6, **characterised in that** R4 stands for H or a straight-chain or branched C₂- to C₂₀-alkenyl group having one or more double bonds.

8. Use according to one or more of Claims 1 to 7, **characterised in that** R3 stands for a radical selected from
- H,
- straight-chain or branched C₁- to C₂₀-alkyl group,
- straight-chain or branched C₂- to C₂₀-alkenyl group having one or more double bonds, where the alkenyl group may also be substituted by one or more saturated or unsaturated cyclohexyl groups,
- an acyl radical of the formula -C(=O)-R6.

9. Use according to one or more of Claims 1 to 8, **characterised in that** the radicals R5 are selected, independently of one another, from H, OH or straight-chain or branched O-(C₁- to C₆-alkyl).

10. Use according to one or more of Claims 1 to 9, **characterised in that** the compound of the formula (I) is selected from the compounds of the formula (Ia) to (Ij)

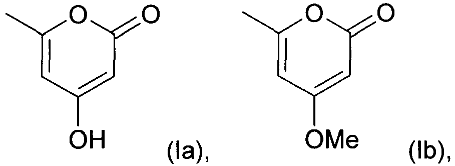

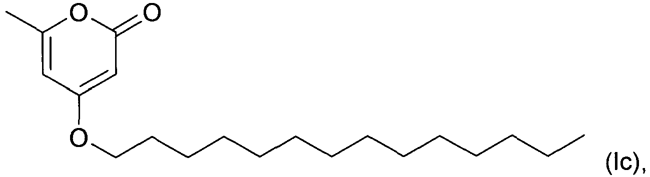

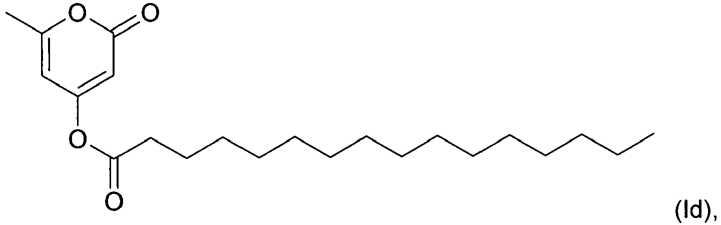

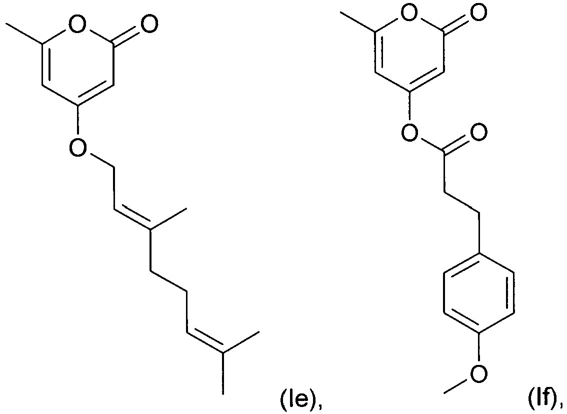

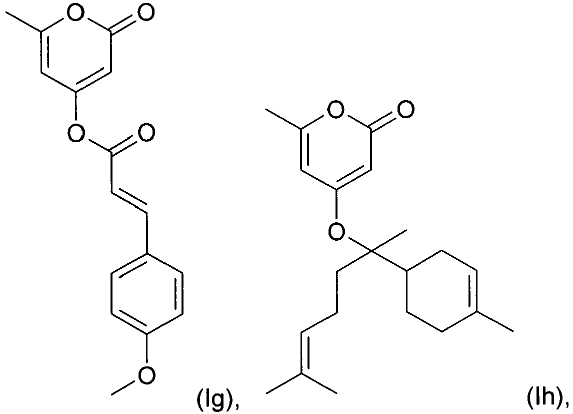

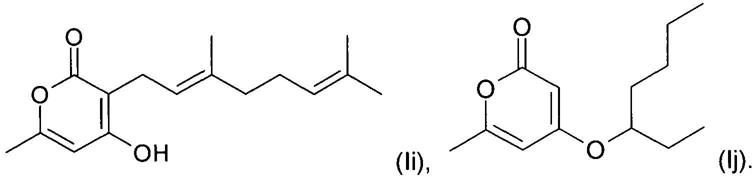

11. Cosmetic or pharmaceutical preparation or food comprising at least one compound of the formula (I) 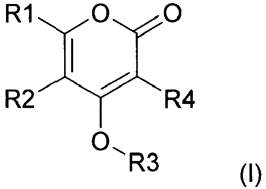 where
R1 stands for a straight-chain or branched C₁- to C₂₀-alkyl group,
R2 stands for
- H,
R4 stands for
- H,
- straight-chain or branched C₁- to C₂₀-alkyl group or
- straight-chain or branched C₂- to C₂₀-alkenyl group having one or more double bonds,
R3 stands for a radical selected from
- H,
- straight-chain or branched C₁- to C₂₀-alkyl group,
- straight-chain or branched C₂- to C₂₀-alkenyl group having one or more double bonds, where the alkenyl group may also be substituted by one or more saturated or unsaturated C₃- to C₁₂cycloalkyl groups,
- straight-chain or branched C₂- to C₂₀-alkynyl group having one or more triple bonds,
- saturated or unsaturated C₃- to C₁₂-cycloalkyl group, where the rings may in each case also be bridged by -(CH₂)ₙ- groups where n = 1 to 3,
- an acyl radical of the formula -C(=O)-R6,
R6 stands for
- straight-chain or branched C₁- to C₂₀-alkyl group,
- a radical of the formula (II) 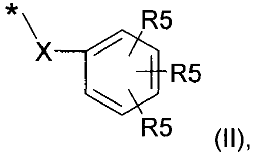 in which X stands for straight-chain or branched C₁- to C₆-alkylene or straight-chain or branched C₂- to C₆-alkenylene and the radicals R5 are selected, independently of one another, from H, OH, straight-chain or branched C₁- to C₆-alkyl or straight-chain or branched O-(C₁- to C₆-alkyl),
and at least one vehicle which is suitable for topical applications or for foods.

12. Preparation according to Claim 11, **characterised in that** the at least one compound of the formula (I) is present in an amount of 0.01 to 20% by weight.

13. Process for preparing a preparation according to Claim 11 or 12, **characterised in that** the compound of the formula (I) is mixed with a vehicle which is suitable for topical applications or for foods.

14. Compounds of the formulae (Id) to (Ij)

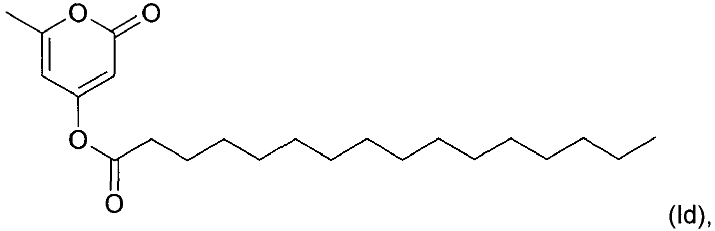

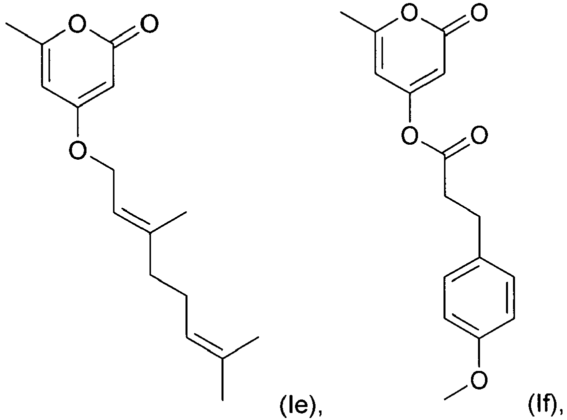

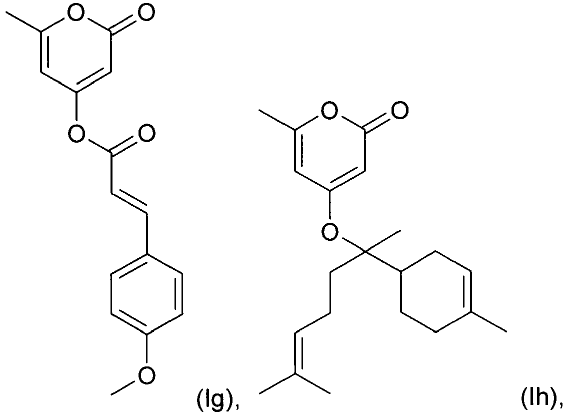

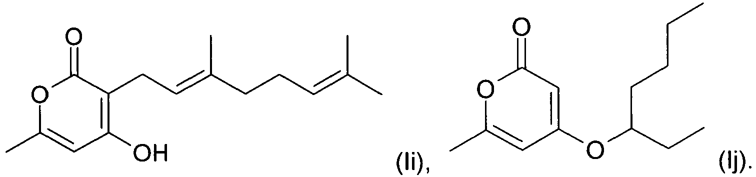

## Revendications

1. Utilisation non thérapeutique d'au moins un composé de formule (I) 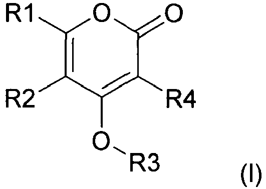 dans lequel
R1 représente un groupement C₁- à C₂₀-alkyle à chaîne linéaire ou ramifiée,
R2 représente
- H,
R4 représente
- H,
- un groupement C₁- à C₂₀-alkyle à chaîne linéaire ou ramifiée ou
- un groupement C₂- à C₂₀-alcényle à chaîne linéaire ou ramifiée ayant une ou plusieurs doubles liaisons,
R3 représente un radical choisi parmi
- H,
- un groupement C₁- à C₂₀-alkyle à chaîne linéaire ou ramifiée,
- un groupement C₂- à C₂₀-alcényle à chaîne linéaire ou ramifiée ayant une ou plusieurs doubles liaisons, où le groupement alcényle peut également être substitué par un ou plusieurs groupements C₃- à C₁₂-cycloalkyle saturés ou insaturés,
- un groupement C₂- à C₂₀-alcynyle à chaîne linéaire ou ramifiée ayant une ou plusieurs triples liaisons,
- un groupement C₃- à C₁₂-cycloalkyle saturé ou insaturé, où les cycles peuvent dans chaque cas être également pontés par des groupements -(CH₂)ₙ- où n = 1 à 3,
- un radical acyle de formule -C(=O)-R6,
R6 représente
- un groupement C₁- à C₂₀-alkyle à chaîne linéaire ou ramifiée,
- un radical de formule (II) 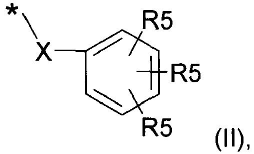 où X représente C₁- à C₆-alkylène à chaîne linéaire ou ramifiée ou C₂- à C₆-alcénylène à chaîne linéaire ou ramifiée et les radicaux R5 sont choisis, indépendamment les uns des autres, parmi H, OH, C₁- à C₆-alkyle à chaîne linéaire ou ramifiée ou O-(C₁- à C₆-alkyl) à chaîne linéaire ou ramifiée,
pour le soin, la conservation ou l'amélioration de l'état général ou de l'aspect de la peau ou des cheveux.

2. Utilisation selon la revendication 1, pour la prévention, la réduction ou la lutte contre la cellulite ou les signes de cellulite et/ou pour la réduction de l'accumulation locale de graisse.

3. Utilisation selon la revendication 1, pour la prophylaxie ou la lutte contre les processus de vieillissement, induits par le temps et/ou la lumière, de la peau ou des cheveux.

4. Utilisation selon la revendication 1 ou 3, pour la prophylaxie contre ou la réduction des irrégularités de la peau, telles que les rides, les ridules, une peau rêche ou une peau à larges pores.

5. Utilisation selon la revendication 2, pour la stimulation de la lipolyse.

6. Utilisation selon l'une ou plusieurs parmi les revendications 1 à 5, **caractérisée en ce que** R1 représente un groupement C₁- à C₆-alkyle à chaîne linéaire ou ramifiée.

7. Utilisation selon l'une ou plusieurs parmi les revendications 1 à 6, **caractérisée en ce que** R4 représente H ou un groupement C₂- à C₂₀-alcényle à chaîne linéaire ou ramifiée ayant une ou plusieurs doubles liaisons.

8. Utilisation selon l'une ou plusieurs parmi les revendications 1 à 7, **caractérisée en ce que** R3 représente un radical choisi parmi
- H,
- un groupement C₁- à C₂₀-alkyle à chaîne linéaire ou ramifiée,
- un groupement C₂- à C₂₀-alcényle à chaîne linéaire ou ramifiée ayant une ou plusieurs doubles liaisons, où le groupement alcényle peut également être substitué par un ou plusieurs groupements cyclohexyle saturés ou insaturés,
- un radical acyle de formule -C(=O)-R6.

9. Utilisation selon l'une ou plusieurs parmi les revendications 1 à 8, **caractérisée en ce que** les radicaux R5 sont choisis, indépendamment les uns des autres, parmi H, OH ou O-(C₁- à C₆-alkyl) à chaîne linéaire ou ramifiée.

10. Utilisation selon l'une ou plusieurs parmi les revendications 1 à 9, **caractérisée en ce que** le composé de formule (I) est choisi parmi les composés de formule (Ia) à (Ij)

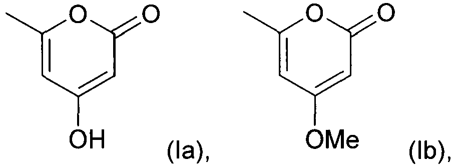

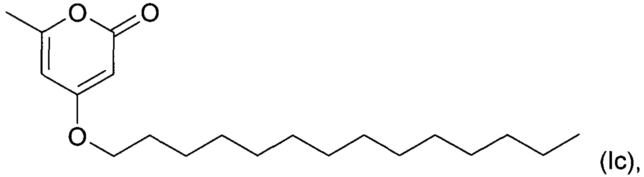

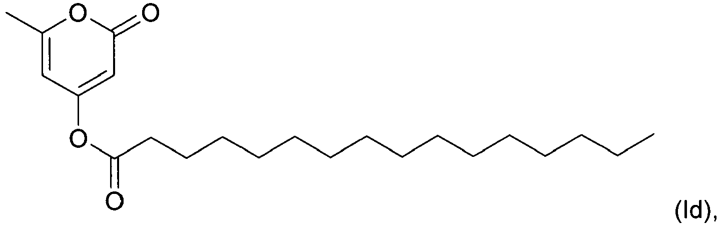

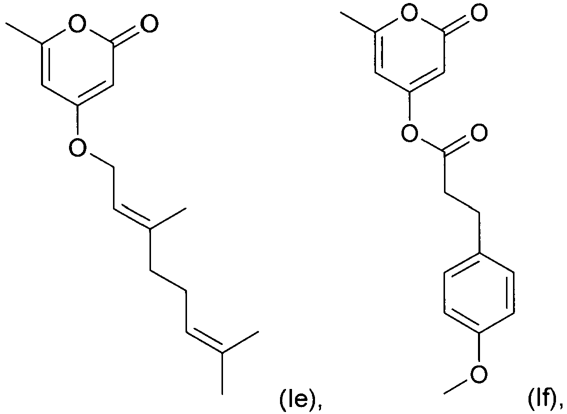

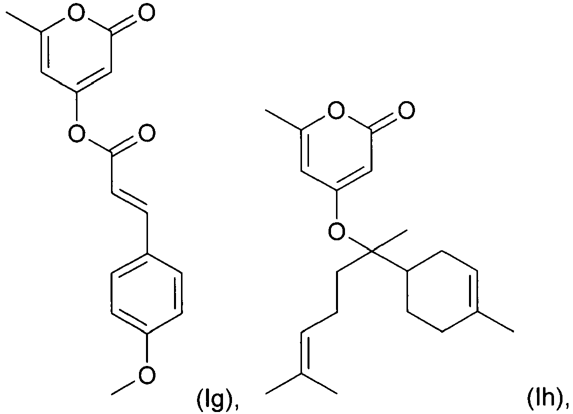

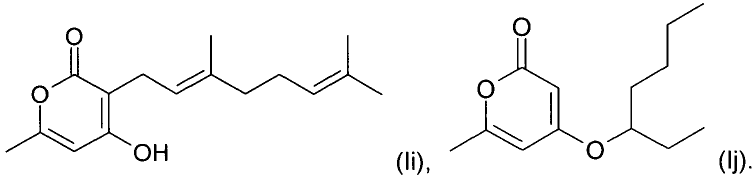

11. Préparation cosmétique ou pharmaceutique ou produit alimentaire, comprenant au moins un composé de formule (I) 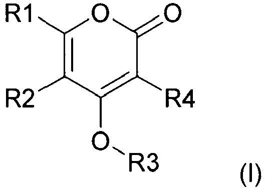 dans lequel
R1 représente un groupement C₁- à C₂₀-alkyle à chaîne linéaire ou ramifiée,
R2 représente
- H,
R4 représente
- H,
- un groupement C₁- à C₂₀-alkyle à chaîne linéaire ou ramifiée ou
- un groupement C₂- à C₂₀-alcényle à chaîne linéaire ou ramifiée ayant une ou plusieurs doubles liaisons,
R3 représente un radical choisi parmi
- H,
- un groupement C₁- à C₂₀-alkyle à chaîne linéaire ou ramifiée,
- un groupement C₂- à C₂₀-alcényle à chaîne linéaire ou ramifiée ayant une ou plusieurs doubles liaisons, où le groupement alcényle peut également être substitué par un ou plusieurs groupements C3- à C₁₂-cycloalkyle saturés ou insaturés,
- un groupement C₂- à C₂₀-alcynyle à chaîne linéaire ou ramifiée ayant une ou plusieurs triples liaisons,
- un groupement C₃- à C₁₂-cycloalkyle saturé ou insaturé, où les cycles peuvent dans chaque cas être également pontés par des groupements -(CH₂)ₙ- où n = 1 à 3,
- un radical acyle de formule -C(=O)-R6,
R6 représente
- un groupement C₁- à C₂₀-alkyle à chaîne linéaire ou ramifiée,
- un radical de formule (II) 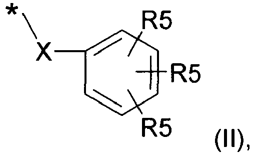 où X représente C₁- à C₆-alkylène à chaîne linéaire ou ramifiée ou C₂- à C₆-alcénylène à chaîne linéaire ou ramifiée et les radicaux R5 sont choisis, indépendamment les uns des autres, parmi H, OH, C₁- à C₆-alkyle à chaîne linéaire ou ramifiée ou O-(C₁- à C₆-alkyl) à chaîne linéaire ou ramifiée,
et au moins un véhicule qui est convenable pour des applications topiques ou pour des produits alimentaires.

12. Préparation selon la revendication 11, **caractérisée en ce que** le au moins un composé de formule (I) est présent selon une quantité allant de 0,01 à 20% en poids.

13. Procédé de préparation d'une préparation selon la revendication 11 ou 12, **caractérisé en ce que** le composé de formule (I) est mélangé avec un véhicule qui est convenable pour des applications topiques ou pour des produits alimentaires.

14. Composés de formules (Id) à (Ij)
